# EUROPEAN PATENT APPLICATION

(11) **EP 1 310 482 A1**
(43) Date of publication of application: **14.05.2003**
(21) Application number: 01500264.5
(22) Date of filing: 07.11.2001
(51) Int. Cl.: C07C 237/22, A61K 31/195, A61P 25/00

(54) **Prodrugs of excitatory amino acids**

(71) Applicant: ELI LILLY AND COMPANY, Indianapolis, Indiana 46285 (US)
(72) Inventor: The designation of the inventor has not yet been filed
(74) Representative: Carpintero Lopez, Francisco

(57) **Abstract**

This invention relates to synthetic excitatory amino acid prodrugs of the formula I 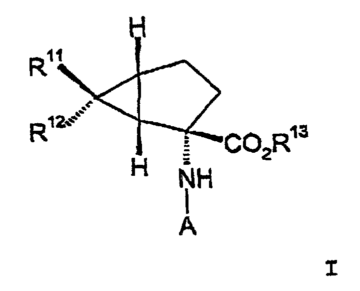 wherein
R¹¹ is CO₂R¹⁴ and R¹² is hydrogen or fluoro; or R¹¹ is hydrogen or fluoro and R¹² is CO₂R¹⁴;
R¹³ and R¹⁴ are, independently, hydrogen, (1-10C) alkyl, (2-4C) alkenyl, aryl or arylalkyl;
A is (Q)ₚ-;
p is any integer from 1-10; and
Q is independently selected, each time taken, from the group amino acyl;
provided that the compound is not one on which R¹¹ is CO₂R¹⁴; R¹², R¹³ and R¹⁴ are hydrogen; p is 1; and Q is L-alanyl;
or a pharmaceutically acceptable salt thereof and processes for their preparation. The invention further relates to methods of using, and pharmaceutical composition comprising, the compounds for the treatment of neurological disorders and psychiatric disorders.

## Description

This invention relates to synthetic excitatory amino acid prodrugs (and their pharmaceutically acceptable salts) and processes for their preparation. The invention further relates to methods of using, and pharmaceutical compositions comprising, the compounds for the treatment of neurological disorders and psychiatric disorders.

Treatment of neurological or psychiatric disorders, such as anxiety disorder, have been linked to selective activation of metabotropic excitatory amino acid receptors such as (+)-2-aminobicyclo[3.1.0]hexane-2,6-dicarboxylic acid, also known as LY354740, which is disclosed in U.S. Patent No. 5,750,566 (the '566 patent) issued May 12, 1998 is an active mGluR2 receptor agonist. CNS Drug Reviews, 5, pgs. 1-12 (1999).

The present invention provides for a prodrug form of LY354740 which enhances the oral exposure of LY354740. The present invention also provides for prodrug forms of other compounds which possess improved oral exposure. Compounds of the present invention represent an improved approach for maintaining LY354740-like safety and efficacy in humans with increased oral bioavailability. Preclinical studies with compounds of the present invention have shown greatly enhanced oral exposure of the parent compound.

Accordingly, the present invention provides a compound of the formula I 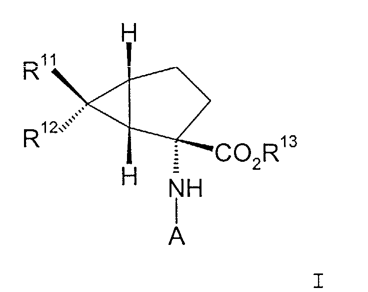 wherein
R¹¹ is CO₂R¹⁴ and R¹² is hydrogen or fluoro; or R¹¹ is hydrogen or fluoro and R¹² is CO₂R¹⁴;
R¹³ and R¹⁴ are independently hydrogen, (1-10C) alkyl, (2-4C) alkenyl, aryl or arylalkyl;
A is (Q)ₚ- ;
p is any integer from 1-10; and
Q is independently selected, each time taken, from the group amino acyl;
provided that the compound is not one in which R¹¹ is CO₂R¹⁴; R¹², R¹³ and R¹⁴ are hydrogen; p is 1 and Q is L-alanyl;
or a pharmaceutically acceptable salt thereof.

Compounds of the present invention have been found to be useful prodrugs for selective agonists of metabotropic glutamate receptors and are therefore useful in the treatment of diseases of the central nervous system such as neurological diseases, for example neurodegenerative diseases, and as antipsychotic, anxiolytic, drug-withdrawal, antidepressant, anticonvulsant, analgesic and anti-emetic agents.

It will be appreciated that the compounds of formula (I) contain at least four asymmetric carbon atoms, three being in the cyclopropane ring and one being at the α-carbon of the amino acid group within the cyclopentane ring. Additional asymmetric carbons may be present in the generic radicals as defined. Accordingly, the compounds of the invention may exist in and be isolated in enantiomerically pure form, in racemic form, or in a diastereoisomeric mixture.

The amino acid moiety within the cyclopentane ring preferably has the natural amino acid configuration, i.e. the L-configuration relating to D-glyceraldehyde.

The present invention includes pharmaceutically acceptable salts of the compound of formula I. These salts can exist in conjunction with the acidic or basic portion of the molecule and can exist as acid addition, primary, secondary, tertiary, or quaternary ammonium, alkali metal, or alkaline earth metal salts. Generally, the acid addition salts are prepared by the reaction of an acid with a compound of formula I. The alkali metal and alkaline earth metal salts are generally prepared by the reaction of the hydroxide form of the desired metal salt with a compound of formula I.

Acids commonly employed to form such salts include inorganic acids, for example hydrochloric, hydrobromic, nitric, sulphuric or phoshoric acids, or with organic acids, such as organic carboxylic acids, for example, glycollic, maleic, hydroxymaleic, fumaric, malic, tartaric, citric, salicyclic, o-acetoxybenzoic, or organic sulphonic acids, for example, 2-hydroxyethane sulphonic, toluene-p-sulphonic, methane-sulfonic or naphthalene-2-sulphonic acid.

In addition to pharmaceutically-acceptable salts, other salts are included in the invention. They may serve as intermediates in the purification of compounds or in the preparation of other, for example pharmaceutically-acceptable, acid addition salts, or are useful for identification, characterization or purification.

A variety of physiological functions have been shown to be subject to influence by excessive or inappropriate stimulation of excitatory amino acid transmission. The compounds of formula I of the present invention are believed to have the ability to treat a variety of neurological disorders in mammals associated with this condition, including acute neurological disorder such as cerebral deficits subsequent to cardiac bypass surgery and grafting, stroke, cerebral ischemia, spinal cord trauma, head trauma, perinatal hypoxia, cardiac arrest, and hypoglycemic neuronal damage. The compounds of formula I are believed to have the ability to treat a variety of chronic neurological disorders, such as Alzheimer's disease, Huntington's Chorea, amyotrophic lateral sclerosis, AIDS-induced dementia, ocular damage and retinopathy, cognitive disorders, and idiopathic and drug-induced Parkinson's. The present invention also provides methods for treating these disorders which comprises administering to a patient in need thereof an effective amount of a compound of formula I or a pharmaceutically acceptable salt thereof.

The compounds of formula I of the present invention treat a variety of other neurological disorders in patients that are associated with glutamate dysfunction, including muscular spasms, convulsions, migraine headaches, urinary incontinence, pain, premenstrual dysphoric disorder (PDD), psychosis, (such as schizophrenia), drug tolerance and withdrawal (such as nicotine, opiates and benzodiazepines), anxiety and related disorders, emesis, brain edema, chronic pain, and tardive dyskinesia. The compounds of formula I are also useful as antidepressant and analgesic agents. Therefore, the present invention also provides methods for treating these disorders which comprise administering to a patient in need thereof an effective amount of the compound of formula I, or a pharmaceutically acceptable salt thereof.

A compound of formula I may be made by a process which is analogous to one known in the chemical art for the production of structurally analogous heterocyclic compounds or by a novel process described herein. Such processes and intermediates useful for the manufacture of a compound of formula I as defined above are provided as further features of the invention and are illustrated by the following procedures in which, unless otherwise specified, the meanings of the generic radicals are as defined above.
(A) For a compound of formula I in which R¹³ and R¹⁴ are hydrogen, deprotecting a compound of formula IV 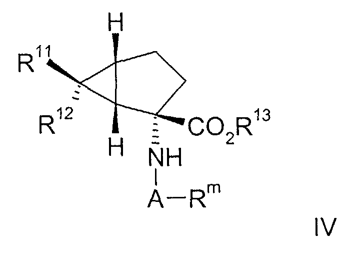 where R^{m} is an amine protecting group and R¹³ and R¹⁴ are carboxy protecting groups as described in the General Procedures for Examples 22-42.
(B) For a compound of formula I in which R¹³ and R¹⁴ are not both hydrogen, acylating a compound of formula II 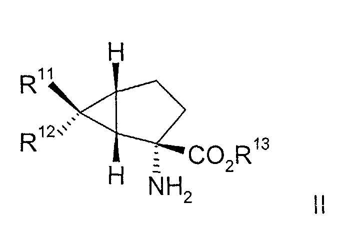 with a corresponding amino acyl of formula III.

   R^{m}A- III

   in which A is (Q)ₚ-, p is any integer from 1-10 and R^{m} is an amine protecting group such as tert-butoxycarbonyl as described in the General Procedures for Examples 1-21.
(C) For a compound of formula II where R¹³ and R¹⁴ are not hydrogen, esterifying a compound of formula II where R¹³ and R¹⁴ are both hydrogen (a di-acid).

The term "amine protecting group," as used herein, refers to those groups intended to protect or block the amine group against undesirable reactions during synthetic procedures. Choice of the suitable amine protecting group used will depend upon the conditions that will be employed in subsequent reaction steps wherein protection is required, as is well within the knowledge of one of ordinary skill in the art. Commonly used amine protecting groups are disclosed in T.W. Greene and P.G.M. Wuts, Protective Groups In Organic Synthesis, 3rd Ed. (John Wiley & Sons, New York (1999)). The amine protecting group is decomposed by using a conventional procedure which does not affect another portion of the molecule.

The term "carboxy protecting group" as used herein refers to one of the ester derivatives of the carboxylic acid group commonly employed to block or protect the carboxylic acid group while reactions are carried out on other functional groups of the compound. Particular values of carboxy protecting group include, for example, methyl, ethyl, tert-butyl, benzyl, methoxymethyl, trimethylsilyl, and the like. Further examples of such groups as well as carboxy-protecting groups may be found in T.W. Greene and P.G.M. Wuts, Protecting Groups in Organic Synthesis, 3rd. Ed. (John Wiley & Sons, N.Y. (1999)). The ester is decomposed by using a conventional procedure which does not affect another portion of the molecule.

Whereafter, for any of the above procedures, when a pharmaceutically acceptable salt of a compound of formula I is required, it is obtained by reacting the acid of formula I with a physiologically acceptable base or by reacting a basic compound of formula I with a physiologically acceptable acid or by any other conventional procedure.

The term "(1-10C) alkyl" represents a straight, branched, or cyclic alkyl chain having from one to ten carbon atoms. Typical straight or branched (1-10C) alkyl groups include methyl, ethyl, *n*-propyl, isopropyl, *n*-butyl, isobutyl, sec-butyl, *t*-butyl, *n*-pentyl, isopentyl, neopentyl, *n*-hexyl, 2-methylpentyl, 3-methylpentyl, 4-methylpentyl, 2,2-dimethylbutyl, 2,3-dimethylbutyl, 3,3-dimethylbutyl, heptyl, *n*-octyl, 2,2-dimethylhexyl, 2,5-dimethylhexyl, 2-methylheptyl, 4-methylheptyl, 2,2,4-trimethylpentyl, 2,3,4-trimethylpentyl, nonyl, 3,5,5-trimethylhexyl, decyl, 3,7-dimethyloctyl, and the like. Typical cyclic alkyl groups include cyclopropyl, cyclobutyl, cyclopentyl, cyclohexyl, cycloheptyl, and the like. The term "(1-10C) alkyl" includes within it the terms "(1-6C) alkyl" and "(1-4C) alkyl". Typical (1-6C) alkyl groups include methyl, ethyl, *n*-propyl, isopropyl, *n*-butyl, isobutyl, sec-butyl, *t*-butyl, *n*-pentyl, isopentyl, neopentyl, and *n*-hexyl. Preferred (1-10C) alkyl groups include methyl.

The term "(2-4C) alkenyl" represents straight or branched unsaturated alkyl chains having from two to four carbon atoms, and having one or more carbon-carbon double bond, such as, dienes. Examples of (2-4C) alkenyl include allyl.

The term "aryl" represents groups such as phenyl, substituted phenyl, and naphthyl. The term "arylalkyl" represents a (1-4C) alkyl group bearing one or more aryl groups. Examples of arylalkyl groups include benzyl.

The term "amino acyl" means an amino acyl derived from an amino acid selected from the group consisting of natural and unnatural amino acids as defined herein. The natural amino acids may be neutral, positive or negative depending on the substituents in the side chain. "Neutral amino acid" means an amino acid containing uncharged side chain substituents. Exemplary neutral amino acids include alanine, valine, leucine, isoleucine, proline, phenylalanine, tryptophan, methionine, glycine, serine, threonine and cysteine. "Positive amino acid" means an amino acid in which the side chain substituents are positively charged at physiological pH. Exemplary positive amino acids include lysine, arginine and histidine. "Negative amino acid" means an amino acid in which the side chain substituents bear a net negative charge at physiological pH. Exemplary negative amino acids include aspartic acid and glutamic acid. Preferred amino acids are α-amino acids. The most preferred amino acids are α-amino acids having L stereochemistry at the α-carbon. Exemplary natural α-amino acids are valine, isoleucine, proline, phenylalanine, tryptophan, methionine, glycine, serine, threonine, cysteine, tyrosine, asparagine, glutamine, lysine, arginine, histidine, aspartic acid and glutamic acid.

"Unnatural amino acid" means an amino acid for which there is no nucleic acid codon. Examples of unnatural amino acids include, for example, the D-isomers of the natural α-amino acids as indicated above; Aib (aminobutyric acid), βAib (3-aminoisobutyric acid), Nva (norvaline), β-Ala, Aad (2-aminoadipic acid), βAad (3-aminoadipic acid), Abu (2-aminobutyric acid), Gaba (γ-aminobutyric acid), Acp (6-aminocaproic acid), Dbu (2,4-diaminobutryic acid), α-aminopimelic acid, TMSA (trimethylsilyl-Ala), aIle (alloisoleucine), Nle (norleucine), *tert*-Leu, Cit (citrulline), Orn, Dpm (2,2'-diaminopimelic acid), Dpr (2,3-diaminopropionic acid), α- or β-Nal, Cha (cyclohexyl-Ala), hydroxyproline, Sar (sarcosine), O-methyl tyrosine, phenyl glycine and the like; cyclic amino acids; N^{a}-alkylated amino acids where N^{a}-alkylated amino acid is N^{a}-(1-10C)alkyl amino acid such as MeGly (N^{a}-methylglycine), EtGly (N^{a}-ethylglycine) and EtAsn (N^{a}-ethylasparagine) and amino acids in which the α-carbon bears two side-chain substituents. Exemplary unnatural α-amino acids include D-alanine, D-leucine and phenylglycine. The names of natural and unnatural amino acids and residues thereof used herein follow the naming conventions suggested by the IUPAC-IUB Joint Commission on Biochemical Nomenclature (JCBN) as set out in "Nomenclature and Symbolism for Amino Acids and Peptides (Recommendations, 1983)" European Journal of Biochemistry, 138, 9-37 (1984). To the extent that the names and abbreviations of amino acids and residues thereof employed in this specification and appended claims differ from those noted, differing names and abbreviations will be made clear.

While all the compounds of formula I of the present invention are believed to provide improved oral exposure, certain compounds of the invention are preferred for such use. Preferably, R¹¹ is CO₂R¹⁴; R¹², R¹³ and R¹⁴ are hydrogen; p is 1-3; and Q is independently selected, each time taken, from L-alanyl, glycyl, L-leucyl, L-phenylalanyl, L-valyl, L-lysyl, L-tryptophyl, L-isoleucyl, L-methionyl, L-glutamyl, L-tyrosyl, D-alanyl, L-prolyl, L-serinyl, D-leucyl, L-asparagyl and L-threonyl. Representative compounds from this preferred group of formula I compounds include:
a) (1*S*,2*S*,5*R*,6*S*)-2-[(2'*S*)-(2'-Amino)-3'-phenylpropionyl]amino-bicyclo[3.1.0]hexane-2,6-dicarboxylic acid hydrochloride;
b) (1*S*,2*S*,5*R*,6*S*)-2-[(2'*S*)-(2'-Amino)-3'-methylbutyryl]amino-bicyclo[3.1.0]hexane-2,6-dicarboxylic acid hydrochloride;
c) (1*S*,2*S*,5*R*,6*S*)-2-[(2'*S*,3'*S*)-(2'-Amino-3'-methylpentanoylamino)-bicyclo[3.1.0]hexane-2,6-dicarboxylic acid hydrochloride;
d) (1*S*,2*S*,5*R*,6*S*)-2-(2-Amino-acetylamino)-bicyclo[3.1.0]hexane-2,6-dicarboxylic acid;
e) (1*S*,2*S*,5*R*,6*S*)-2-[(2'*S*)-(2'-Amino)-(4'-methylthio)-butyryl]amino-bicyclo[3.1.0]hexane-2,6-dicarboxylic acid;
f) (1*S*,2*S*,5*R*,6*S*)-2-[(2'*S*)-(2'-amino)-(3'-*p*-hydroxyphenyl)-propionyl]amino-bicyclo[3.1.0]hexane-2,6-dicarboxylic acid;
g) (1*S*,2*S*,5*R*,6*S*)-2-[(2'*S*,3'*R*)-2-amino-3-hydroxy)-butyryl)amino-bicyclo[3.1.0]hexane-2,6-dicarboxylic acid;
h) (1*S*,2S,5R,6*S*)-2-[2'S-(2"S-amino-4-methylpentanonylamino)-propionylamino]-bicyclo[3.1.0]hexane-2,6-dicarboxylic acid; and
i) (1*S*,2*S*,5R,6*S*)-2-[2'(S)-(2"(S)-amino-propionylamino)-propionylamino]-bicyclo[3.1.0]hexane-2,6-dicarboxylic acid.

More preferred compounds of formula I are where p is 1. Representative compounds from this more preferred group of formula I compounds include (1*S*,2*S*,5*R*,6*S*)-2-[(2'*S*)-(2'-amino-4'-methyl)-pentanoyl]amino-bicyclo[3.1.0]hexane-2,6-dicarboxylic acid.

The term "affecting" refers to a formula I compound acting as an agonist at an excitatory amino acid receptor. The term "excitatory amino acid receptor" refers to a metabotropic glutamate receptor, a receptor that is coupled to cellular effectors via GTP-binding proteins. The term "cAMP-linked metabotropic glutamate receptor" refers to a metabotropic receptor that is coupled to inhibition of adenylate cyclase activity.

The term "neurological disorder" refers to both acute and chronic neurodegenerative conditions, including cerebral deficits subsequent to cardiac bypass surgery and grafting, cerebral ischemia (for example stroke resulting from cardiac arrest), spinal cord trauma, head trauma, Alzheimer's Disease, Huntington's Chorea, amyotrophic lateral sclerosis, AIDS-induced dementia, perinatal hypoxia, hypoglycemic neuronal damage, ocular damage and retinopathy, cognitive disorders, idiopathic and drug-induced Parkinson's Disease. This term also includes other neurological conditions that are caused by glutamate dysfunction, including muscular spasms, migraine headaches, urinary incontinence, drug tolerance, withdrawal, and cessation (i.e. opiates, benzodiazepines, nicotine, cocaine, or ethanol), smoking cessation, emesis, brain edema, chronic pain, sleep disorders, convulsions, Tourette's syndrome, attention deficit disorder, and tardive dyskinesia.

The term "psychiatric disorder" refers to both acute and chronic psychiatric conditions, including schizophrenia, anxiety and related disorders (e.g. panic attack and stress-related cardiovascular disorders), depression, bipolar disorders, psychosis, and obsessive compulsive disorders.

A used herein the term "effective amount" refers to the amount or dose of the compound, upon single or multiple dose administration to the patient, which provides the desired effect in the patient under diagnosis or treatment.

An effective amount can be readily determined by the attending diagnostician, as one skilled in the art, by the use of known techniques and by observing results obtained under analogous circumstances. In determining the effective amount or dose of compound administered, a number of factors are considered by the attending diagnostician, including, but not limited to: the species of mammal; its size, age, and general health; the spe4cific disease involved; the degree of or involvement or the severity of the disease; the response of the individual patient; the particular compound administered; the mode of administration; the bioavailability characteristics of the preparation administered; the dose regimen selected; the use of concomitant medication; and other relevant circumstances. For example, a typical daily dose may contain from about 25 mg to about 300 mg of the active ingredient. The compounds can be administered by a variety of routes including oral, rectal, transdermal, subcutaneous, intravenous, intramuscular, bucal or intranasal routes. Alternatively, the compound may be administered by continuous infusion.

As used herein the term "patient" refers to a mammal, such as a mouse, guinea pig, rat, dog or human. It is understood that the preferred patient is a human.

The term "treating" (or "treat") as used herein includes its generally accepted meaning which encompasses prohibiting, preventing, restraining, and slowing, stopping, or reversing progression of a resultant symptom. As such, the methods of this invention encompass both therapeutic and prophylactic administration.

If not commercially available, the necessary starting materials for the above procedures may be made by procedures which are selected from standard techniques of organic and heterocyclic chemistry, techniques which analogous to the syntheses of known, structurally similar compounds, and the procedures described in the Examples, including novel procedures.

A further aspect of the present invention provides for a method of administering an effective amount of a compound of formula II, where R¹³ and R¹⁴ are both hydrogen (a di-acid), which comprises administering to a patient requiring modulated excitatory amino acid neurotransmission a pharmaceutically-effective amount of a compound of formula I.

Compounds of formula I are converted via enzymatic or hydrolytic process in vivo, to form compounds of formula II, where R¹³ and R¹⁴ are both hydrogen (a di-acid).

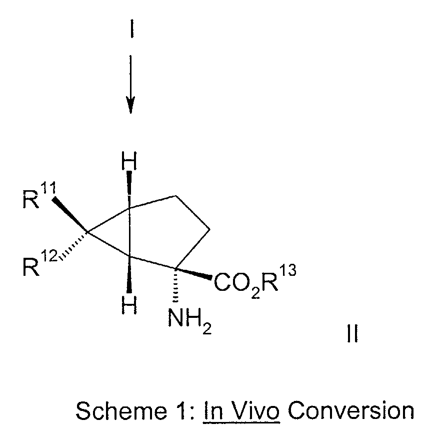

A crystalline form of a compound of formula I may be prepared according to the route outlined in Scheme 2 below in which each R¹³ and R¹⁴, respectively, represents a value defined for the groups R¹³ and R¹⁴. The process described in Scheme 2 is a synthesis method for the preparation of a crystalline hydrochloride form of a compound of formula I and a methanesulfonate salt form of a compound of formula I.

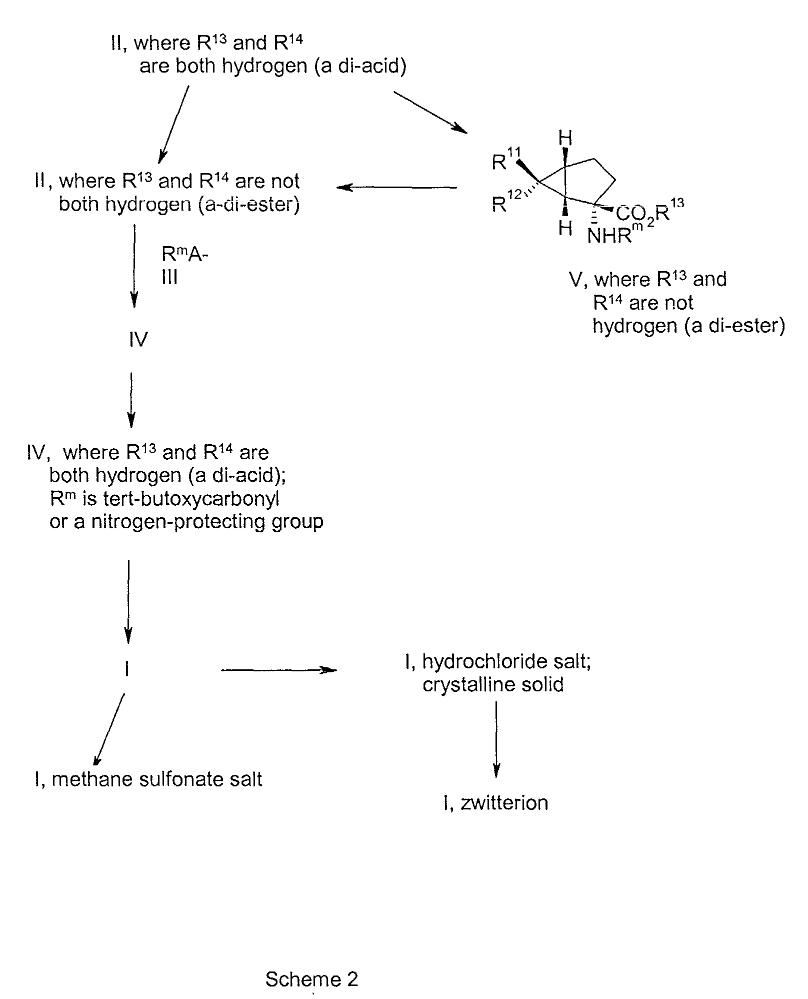

In scheme 2 above, the monohydrate of II, where R¹³ and R¹⁴ are both hydrogen (a di-acid), is treated with carboxy protecting agents such as thionyl chloride or hydrochloric acid in methanol to afford the corresponding di-ester of II.

Alternatively, compound II is treated with amine protecting agents such as di-tert-butyl dicarbonate in a suitable base such as sodium hydroxide to afford compound V where R¹³ and R¹⁴ are both hydrogen. Convenient solvents include tetrahydrofuran.

Compound V is treated with carboxy protecting agents such as methyl iodide in a suitable base such as potassium carbonate to afford compound V where R¹³ and R¹⁴ are both carboxy protecting groups. Convenient solvents include dimethylforamide. Compound V is treated with amine deprotecting agents such as hydrochloric acid to afford the diester compound of formula II where R¹³ and R¹⁴ are both carboxy protecting groups. Convenient solvents include ethyl acetate.

The di-ester, formula II, is amidated with a compound of formula III using dicyclohexylcarbodiimide (DCC), 1-(3-dimethylaminopropyl)-3-ethylcarbodiimide (EDCI) or isobutyl chloroformate as coupling agents to afford a di-ester protected peptidyl compound of formula I. This transformation could also be achieved using the acid chloride or by using a variety of other peptide coupling reagents, for example, diphenyl chlorophosphate and 2-chloro-4,6-dimethoxy-1,3,5-triazine (CDMT), bis(2-oxo-3-oxazolidinyl)phosphinic chloride and benzotriazol-1-yloxytris(dimethylamino)phosphonium hexafluorophosphate.

The hydrolysis of the di-ester protected peptidyl compound of formula I with a suitable base such as lithium hydroxide or sodium hydroxide in THF affords the di-acid protected peptidyl compound of formula I, where R¹³ and R¹⁴ are both hydrogen (a di-acid). The di-acid amine protected peptidyl compound of formula IV may be deprotected with a mineral or organic acid in a suitable solvent. Such conditions may produce the corresponding acid salt of the di-acid peptidyl compound of formula I as an amorphous solid or, directly, a crystalline solid. In the case of an amorphous solid, subsequent crystallization may occur from suitable solvents. For example, a di-acid protected peptidyl compound of formula IV when treated hydrogen chloride gas in ethyl acetate provides the deprotected hydrochloride salt as an amorphous solid. The amorphous hydrochloride compound may then be crystallized from acetone and water to afford the crystalline hydrochloride salt compound of formula I. In the case of a crystalline solid which is formed directly, filtration of the reaction mixture may afford the crystalline salt. The zwitterionic compound of formula I is afforded by treatment of the crystalline hydrochloride salt of formula I with sodium hydroxide or by purification by HPLC using an appropriate buffered solvent mixture. It will be appreciated by one of ordinary skill in the art that a compound of formula I may be prepared in one procedure where the indicated intermediates are not isolated.

The ability of compounds to modulate metabotropic glutamate receptor function may be demonstrated by examining their ability to influence either cAMP production (mGluR 2, 3, 4, 6, 7 or 8) or phosphoinositide hydrolysis (mGluR 1 or 5) in cells expressing these individual human metabotropic glutamate receptor (mGluR) subtypes. (D. D. Schoepp, *et al*., *Neuropharmacol.,* 1996, 35, 1661-1672 and 1997, 36, 1-11) .

The ability of compounds of formula I to treat anxiety or a related disorder may be demonstrated using the well known fear potentiated startle and elevated plus maze models of anxiety described respectively in Davis, Psychopharmacology, 62:1;1979 and Lister, Psychopharmacol, 92:180-185; 1987

### In Vivo Exposure as Measured by Rat Plasma Concentration

To study the in vivo exposure of LY354740 following oral dosing of compounds of the present invention in comparison to LY354740, studies measuring the plasma concentrations of LY354740 in rats were performed.

Mature Fischer 344 male rats (190-270 gram) were obtained from Harlan Sprague-Dawley, Cumberland, IN, USA and acclimated in the study housing for 3 days. On day 4, test compounds were dissolved in buffered water (1mg/ml = test compound/20mM potassium dihydrogen phosphate, pH=2) and given orally (P. O.) as a single 5mg/kg dose. Blood samples were collected through orbital sinus or cardiac puncture (last time point) at 0.5 and 1 hour or, alternatively, 1 and 3 hours. Plasma samples were stored at -20°C in the presence of phenylmethylsulfonyl fluoride, a protease inhibitor, prior to analysis. Plasma samples and internal standard compounds were pretreated by solid phase extraction (SAX support, methanol/water/dilute acetic acid). As shown in the second row of Table 1, below, the plasma concentrations (ng/ml) of LY354740 for each test compound were determined by LC/MS/MS and are presented as a sum of the concentrations at the 0.5 and 1 hour or, alternatively, 1 and 3 hour sample time points.

**Table 1.**

| **Comparison of plasma concentrations of LY354740 and compounds of the present invention** | |
|---|---|
| **Compound (@5mg/kg p. o.)** | **Plasma Concentration of LY354740, ng/ml (sum of 0.5 and 1 hour)** |
| LY354740 | 466 |
| Example 22 | 7114 |
| Example 23 | 8812 |
| Example 24 | 4037* |
| Example 25 | 4512* |
| Example 27 | 2180 |
| Example 28 | 4041 |
| Example 29 | 4094 |
| Example 30 | 5827 |
| Example 31 | 2334 |
| Example 32 | 4079 |
| Example 35 | 5174 |
| Example 36 | 3652 |
| Example 38 | 2861 |
| Example 39 | 4791 |

| | |
|---|---|
| * sum of 1 and 3 hour | |

The compounds of the present invention are preferably formulated prior to administration. Therefore, another aspect of the present invention is a pharmaceutical formulation comprising a compound of formula I, a pharmaceutically acceptable metabolically labile ester thereof, or a pharmaceutically acceptable salt thereof, and a pharmaceutically-acceptable carrier, diluent, or excipient. The pharmaceutical formulations may be prepared by procedures using well-known by one of ordinary skill in the art. In making the compositions of the present invention, the active ingredient will usually be mixed with a carrier, or diluted by a carrier, or enclosed within a carrier, and may be in the form of a capsule, sachet, paper, or other container. When the carrier serves as a diluent, it may be a solid, semi-solid, or liquid material which acts as a vehicle, excipient, or medium for the active ingredient. The compositions can be in the form of tablets, pills, powders, lozenges, sachets, cachets, elixirs, suspensions, emulsions, solutions, syrups, aerosols, ointments containing, for example, up to 10% by weight of active compound, soft and hard gelatin capsules, suppositories, sterile injectable solutions, and sterile packaged powders.

Some examples of suitable carriers, excipients, and diluents include lactose, dextrose, sucrose, sorbitol, mannitol, starches, gum, acacia, calcium phosphate, alginates, tragacanth, gelatin, calcium silicate, microcrystalline cellulose, polyvinylpyrrolidone, cellulose, water syrup, methyl cellulose, methyl and propyl hydroxybenzoates, talc, magnesium stearate, and mineral oil. The formulations can additionally include lubricating agents, wetting agents, emulsifying and suspending agents, preserving agents, sweetening agents, or flavoring agents. Compositions of the invention may be formulated so as to provide quick, sustained, or delayed release of the active ingredient after administration to the patient by employing procedures well known in the art.

The compositions are preferably formulated in a unit dosage form, each dosage containing from about 5 mg to about 500 mg, more preferably about 25 mg to about 300 mg of the active ingredient. As used herein, the term "active ingredient" refers to a compound included within the scope of formula I.

The term "unit dosage form" refers to a physically discrete unit suitable as unitary dosages for human subjects and other mammals, each unit containing a predetermined quantity of active material calculated to produce the desired therapeutic effect, in association with a suitable pharmaceutical carrier, diluent, or excipient.

The following Examples further illustrate the compounds of the present invention and the methods for their synthesis. The Examples are not intended to be limiting to the scope of the invention in any respect, and should not be so construed. All experiments were run under a positive pressure of dry inert gas such as nitrogen or argon. All solvents and reagents were purchased from commercial sources and used as received, unless otherwise indicated. Dry tetrahydrofuran (THF) was obtained by distillation from sodium or sodium benzophenone ketyl prior to use. Proton nuclear magnetic resonance (¹H NMR) spectra were obtained on a Bruker Avance II bay-500 at 500 MHz, a Bruker Avance I bay-200GE at 200MHz or a Varian Inova at 500 MHz. Electrospray mass spectroscopy (ESI) was performed on a Agilent MSD/B intrument using acetonitrile/aqueous ammonium acetate as a mobile phase. Free atom bombardment mass spectroscopy (FABMS) was performed on a VG ZAB-2SE instrument. Field desorption mass spectroscopy (FDMS) was performed using either a VG 70SE or a Varian MAT 731 instrument. Optical rotations were measured with a Perkin-Elmer 241 polarimeter. Chromatographic separation on a Waters Prep 500 LC was generally carried out using a linear gradient of the solvents indicated in the text. The reactions were generally monitored for completion using thin layer chromatography (TLC). Thin layer chromatography was performed using E. Merck Kieselgel 60 F₂₅₄ plates, 5 cm X 10 cm, 0.25 mm thickness. Spots were detected using a combination of UV and chemical detection (plates dipped in a ceric ammonium molybdate solution [75 g of ammonium molybdate and 4 g of cerium (IV) sulfate in 500 mL of 10% aqueous sulfuric acid] and then heated on a hot plate). Flash or silica gel chromatography was performed as described by Still, *et al*. Still, Kahn, and Mitra, *J. Org. Chem.,* **43,** 2923 (1978). Elemental analyses for carbon, hydrogen, and nitrogen were determined on a Control Equipment Corporation 440 Elemental Analyzer, or were performed by the Universidad Complutense Analytical Centre (Facultad de Farmacia, Madrid, Spain). Melting points were determined in open glass capillaries on a Gallenkamp hot air bath melting point apparatus or a Büchi melting point apparatus, and are uncorrected.

The abbreviations, symbols and terms used in the examples have the following meanings.
Ac = acetyl
Anal. = elemental analysis
Bn or Bzl = benzyl
Bu = butyl
BOC = tert-butoxycarbonyl
calcd = calculated
D₂O = deuterium oxide
DCC = dicyclohexylcarbodiimide
DIBAL-H = diisobutyl aluminum hydride
DMAP = dimethylaminopyridine
DMF = dimethylformamide
DMSO = dimethylsulfoxide
EDC = N-ethyl-N'N'-dimethylaminopropyl carbodiimide
Et = ethyl
EtOH = ethanol
FAB = Fast Atom Bombardment (Mass Spectrascopy)
FDMS = field desorption mass spectrum
HOAt = 1-hydroxy-7-azabenzotriazole
HOBt = 1-hydroxybenzotriazole
HPLC = High Performance Liquid Chromatography
HRMS = high resolution mass spectrum
i-PrOH = isopropanol
IR = Infrared Spectrum
L = liter
Me = methyl
MeOH = methanol
MPLC = Medium Pressure Liquid Chromatography
Mp = melting point
MTBE = t-butyl methyl ether
NBS = N-bromosuccinimide
NMR = Nuclear Magnetic Resonance
Ph = phenyl
p.o. = oral administration
i-Pr = isopropyl
Rochelle's Salt = potassium sodium tartrate
SM = starting material
TBS = tert-butyldimethylsilyl
TEA = triethylamine
Temp. = temperature
TFA = trifluoroacetic acid
THF = tetrahydrofuran
TLC = thin layer chromatography
t-BOC = tert-butoxycarbonyl

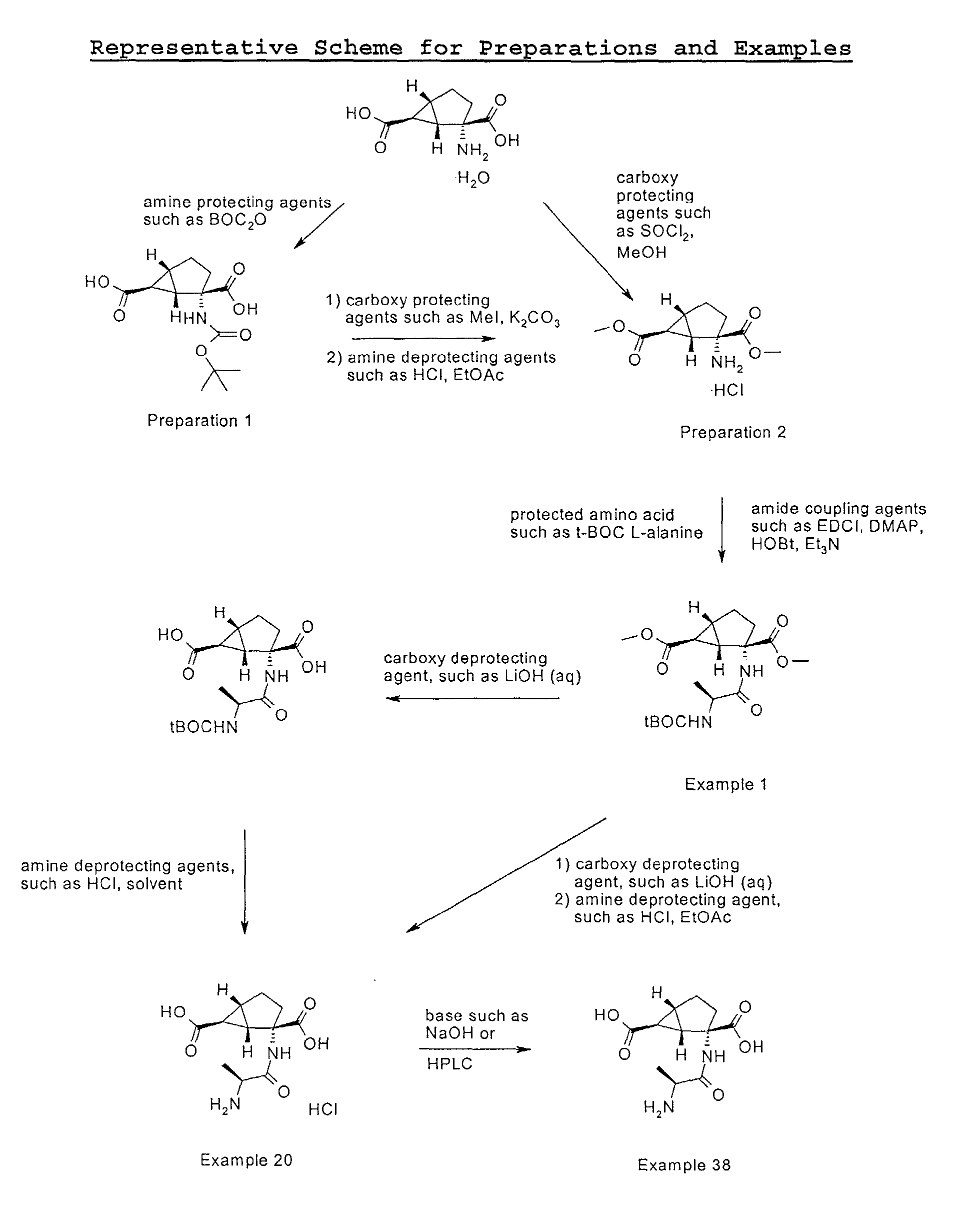

### Preparation 1

### Synthesis of (1S,2S,5R,6S)-2-tert-Butoxycarbonylaminobicyclo[3.1.0]hexane-2,6-dicarboxylic acid

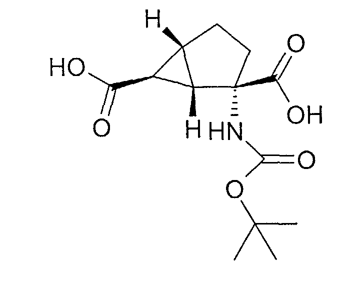

A 1 L flask was charged with (1*S*,2*S*,5*R*,6*S*)-2-aminobicyclo[3.1.0]hexane-2,6-dicarboxylic acid monohydrate (24.4 g, 0.12 mol, 1 equiv), dioxane (200 mL) and di-*tert*-butyl dicarbonate (52.4 g, 0.24 mol, 2.0 equiv). The suspension was vigorously stirred while IN sodium hydroxide (420 mL, 3.5 equiv) was added. The mixture was stirred for 2 days, then 2.0 more equiv of di-*tert*-butyl dicarbonate were added and the reaction stirred for 3 additional days at room temperature. After 5 total days of reaction, water (400 mL) was added to dissolve the salts. The aqueous layer was extracted with ethyl acetate (4 x 100 mL) and acidified to pH 2 with 6 N hydrochloric acid. The acidic aqueous phase was extracted with ethyl ether (6 x 200 mL). The combined ether extracts were washed with water (250 mL) and brine (250 mL). After drying over sodium sulfate, solvents were evaporated under vacuum to afford a foamy white solid (26.4 g).
77% Yield; mp 100-101 °C.
[α]_{D}²⁵ = - 41.1 ° (c = 1.0, MeOH).
¹H NMK (Methanol-*d*₄) δ: 4.98 (brs, 1H), 2.44 (dd, 1H, *J* = 6.2, 2.6 Hz), 2.19-1.92 (m, 4H), 1.62 (t, 1H, *J* = 2.8 Hz), 1.43 (s, 9H), 1.29 (m, 1H).
¹³C NMR (Methanol-*d*₄) δ: 175.6, 175.2, 158.2, 60.1, 34.6, 31.9, 28.4, 27.2, 25.6, 20.6.
MS (Electrospray): 285.12.

### Preparation 2

### Synthesis of (1S,2S,5R,6S)-2-Amino-bicyclo[3.1.0]hexane-2,6-dicarboxylic acid dimethyl ester hydrochloride

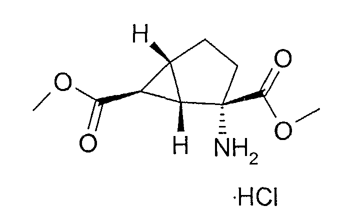

(1*S*,2*S*,5*R*,6*S*)-2-*tert*-Butoxycarbonylaminobicyclo[3.1.0]hexane-2,6-dicarboxylic acid (20 g, 0.07 mol, 1.0 equiv) was dissolved in 210 ml of dry dimethylformamide and potassium carbonate (21.3 g, 0.154 mol, 2.2 equiv) was added at 0 °C under nitrogen. After 15 minutes, methyl iodide (17.6 ml, 0.28 mol, 4.0 equiv) was added. The reaction mixture was warmed up slowly and stirred at room temperature for 3h. Water (200 ml) was added and the aqueous phase was extracted with ethyl ether (4 x 75 ml each). The combined organic phase was washed with cold water (4 x 50 ml), and the aqueous phase extracted again with ethyl ether (2 x 50 ml). After drying the organic phase over sodium sulfate and evaporating under vacuum, a foamy solid ((1*S*,2*S*,5*R*,6*S*)-2-*tert*-butoxycarbonylaminobicyclo[3.1.0]hexane-2,6-dicarboxylic acid-2,6-dimethyl ester) was obtained (19.2 g, 87% yield).

This compound was diluted with 150 ml of a saturated solution of hydrogen chloride gas in ethyl acetate and the mixture vigorously stirred for 1 hour (a white precipitate appeared within 15 minutes). The solid was filtered, rinsed with ethyl ether and thoroughly dried under high vacuum.
73% Yield; mp 193-194 °C.
[α]_{D}²⁵ = + 22.2 ° (c = 1.0, MeOH).
¹H NMR (D₂O) δ: 3.86 (s, 3H), 3.67 (s, 3H), 2.31-2.04 (m, 6H), 1.57 (m, 1H).
¹³C NMR (Methanol-*d*₄) δ: 171.9, 170.2, 65.6, 52.8, 51.2, 32.4, 29.9, 28.5, 26.2, 20.7.

### Alternative Synthesis of (1S,2S,5R,6S)-2-Aminobicyclo[3.1.0]hexane-2,6-dicarboxylic acid dimethyl ester hydrochloride

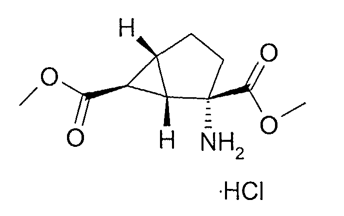

Thionyl chloride (807 mL, 11.1 mol) was added to methanol (9.5 L) over a period of 1 h while maintaining the temperature between 2 - 20 °C. The solution was maintained for 30 min, then (1S,2S,5R,6S)-2-amino-bicyclo[3.1.0]hexane-2,6-dicarboxylic acid monohydrate (1.61 kg, 7.92 mol) was added. The resulting solution was heated to 47 °C and maintained between 47 - 50 °C for 17 h. Approximately 7.3 L of methanol was then removed by vacuum distillation (47 - 50 °C, 240 - 275 mm Hg). The remaining methanol was removed by azeotropic distillation with t-butyl methyl ether (MTBE) at atmospheric pressure [added MTBE (10 L), removed 8.5 L; added MTBE (10 L), removed 8.5 L; added MTBE (8 L), removed 5.1 L]. During the course of the distillations a white solid began to precipitate from the solution. After completion of the distillations, MTBE (2 L) was added to the resulting slurry, and the slurry was cooled to 22 °C. The solid was filtered, rinsed with MTBE (2 L) and dried under vacuum to afford 1.94 kg (98%) of the title compound as a white solid.
Analysis Calculated for C₁₀H₁₆NO₄Cl: C, 48.10; H, 6.46; N, 5.61; Cl, 14.20.;
Found: C, 47.88; H, 6.25; N, 5.57; Cl, 14.52.

### Preparation 3

### Synthesis of 2(S)-(2'(S)-tert-Butoxycarbonylamino-4-methylpentanonylamino)-propionic acid.

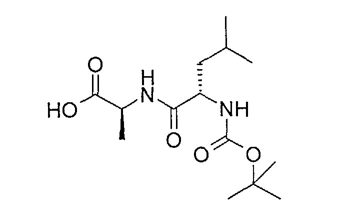

In a flask containing L-leucinyl-L-alanine (5 mmol), di-tert-butyl dicarbonate (10 mmol) dissolved in 15 mL of dioxane and 15 mL of saturated aqueous sodium bicarbonate were added. The reaction was stirred at room temperature overnight. It was diluted with water (100 mL) and washed with EtOAc (2x50 mL). The aqueous layer was acidified to pH 1 with 6N hydrochloric acid and extracted with EtOAc. The organic layer was washed with brine, dried over MgSO₄, filtered and evaporated to provide the title compound. Yield: 78%
¹H NMR (CDCl₃) : 6.90 (bs, 1 H); 5.15 (bs, 1 H); 4.63-4.46 (m, 1 H); 4.17-4.06 (m, 2 H); 1.75-1.43 (m, 15 H); 0.95-0.91 (m, 6 H) .

### Preparation 4

### Synthesis of 2S-[2'S-(2"S-tert-Butoxycarbonylaminopropionyl-amino)-propionylamino]-propionic acid

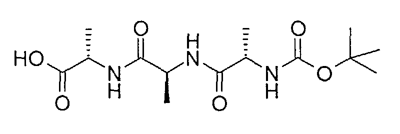

The title compound was prepared according to the procedure of Preparation 3 substituting L-alanyl-L-alanyl-L-alanine for L-leucinyl-L-alanine.
1H NMR (CD3OD): 4.48-4.38 (2 H, m), 4.10-4.03 (1H, m), 1.50 (9H, s), 1.4-1.2 (9H, 3 x d, 7.0 Hz)
MS: m/z 231 [M + H - CO2tBu]

### General Procedure 1

### General Procedure for the coupling reaction of [1S,2S,5R,6S)-2-Amino-bicyclo[3.1.0]hexane-2,6-dicarboxylic acid dimethyl ester hydrochloride with N-BOC-aminoacids.

The starting dimethyl ester hydrochloride salt (1.0 equiv), the product of Preparation 2, was suspended in dry dichloromethane (0.1 M solution) under nitrogen. The corresponding N-BOC-aminoacid (1.3 to 1.5 equiv), N-ethyl-N',N'-dimethylaminopropylcarbodiimide (EDC, 1.4 to 1.5 equiv) and 1-hydroxybenzotriazole (HOBt, 1.2 to 1.5 equiv) were added in one portion, followed by triethyl amine(1.0 to 1.3 equiv) via syringe and, finally, dimethylaminopyridine (DMAP, 0.1 equiv). The reaction mixture was stirred overnight at room temperature, then hydrolyzed by addition of IN hydrochloric acid (20 ml / mmol) and diluted with methylene chloride (10 ml / mmol). The aqueous layer was extracted with methylene chloride (5 ml / mmol) and the combined organic layers washed twice with 1 N hydrochloric acid (10 ml / mmol), and finally with water and brine (10 ml / mmol each). After drying over sodium sulfate and evaporation under vacuum the crude residue was purified by silica gel chromatography using the appropriate eluent for example mixtures hexanes and ethyl acetate.

### Alternative procedure for the coupling reaction of (1S,2S,5R,6S)-2-Amino-bicyclo[3.1.0]hexane-2,6-dicarboxylic acid dimethyl ester hydrochloride with N-BOC-aminoacids.

A solution of dicyclohexylcarbodiimide (DCC) (1.1 equiv) in methylene chloride (4.0 M solution) was added to a mixture of Example Preparation 2 (1.0 equiv), triethylamine (1.0 equiv) and N-t-butoxycarbonyl-L-alanine (1.1 equiv) in methylene chloride (1.0 M solution) over a period of approximately 1.5 h while stirring. The resulting mixture was stirred for 1 - 12 h then filtered. The filter cake (dicyclohexylurea) was rinsed with methylene chloride, and the filtrate was washed with 0.1 M NaHCO₃ followed by 1.0 N hydrochloric acid. The organic phase was dried (Na₂SO₄), filtered and concentrated to afford the title compound as an oil.

### Example 1

### Synthesis of (1S,2S,5R,6S)-2-[(2'S)-(2'-tert-Butoxycarbonylamino)-propionyl]amino-bicyclo[3.1.0]hexane-2,6-dicarboxylic acid dimethyl ester

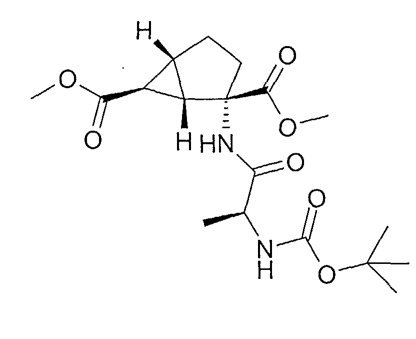

The title compound was prepared using commercially available N-BOC-(L)-alanine according to General Procedure 1.
50% Yield. Foamy white solid. mp 51-52 °C.
[α]_{D}²⁵ = - 27.7 ° (c = 0.52, CHCl₃).
¹H NMR (CDCl₃) δ: 7.28 (brs, 1H), 5.04 (brd, 1H, *J* = 7.6 Hz), 4.16 (m, 1H), 3.74 (s, 3H), 3.66 (s, 3H), 2.49 (dd, 1H, *J* = 13.9, 8.3 Hz), 2.42 (dd, 1H, *J* = 6.3, 2.8 Hz), 2.18-1.89 (m, 3H), 1.70 (t, 1H, *J* = 2.9 Hz), 1.45 (s, 9H), 1.33 (d, 3H, *J* = 7.0 Hz), 1.19 (m, 1H).
¹³C NMR (CDCl₃) δ: 172.8, 172.6, 172.6, 155.7, 80.2, 66.3, 52.6, 51.8, 49.5, 34.4, 32.0, 28.2, 28.1, 26.6, 21.1, 17.6.

### Example 2

### Synthesis of (1S,2S,5R,6S)-2-[(2'S)-(2'-tert-Butoxycarbonylamino-4'-methyl)-pentanoyl]aminobicyclo[3.1.0]hexane-2,6-dicarboxylic acid dimethyl ester

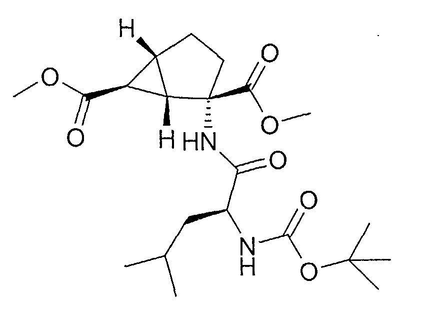

The title compound was prepared using commercially available N-BOC-(*L*)-leucine according to General Procedure 1.
95% Yield. Foamy white solid. mp 62-63 °C.
[α]_{D}²⁵ = - 27.2 ° (c = 1.0, CHCl₃).
¹H NMR (CDCl₃) δ: 7.00 (brs, 1H), 4.97 (brd, 1H, *J* = 8.4), 4.05 (m, 1H), 3.69 (s, 3H), 3.62 (s, 3H), 2.40 (m, 2H), 2.08-1.84 (m, 3H), 1.67-1.58 (m, 3H), 1.41 (m, 1H), 1.41 (s, 9H), 1.22 (m, 1H), 0.90 (d, 3H, *J* = 6.2 Hz), 0.89 (d, 3H, *J* = 6.1 Hz).
¹³C NMR (CDCl₃) δ: 172.8, 172.6, 172.5, 155.8, 80.0, 66.2, 52.5, 51.7, 40.7, 34.3, 31.9, 28.2, 28.1, 26.4, 24.6, 22.7, 22.0, 21.0.

### Example 3

### Synthesis of (1S,2S,5R,6S)-2-[(2'S)-(2'-tert-Butoxycarbonylamino)-3'-phenylpropionyl] aminobicyclo[3.1.0]hexane-2,6-dicarboxylic acid dimethyl ester

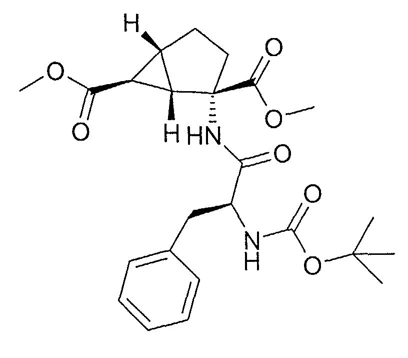

The title compound was prepared using commercially available N-BOC-(*L*)-phenylalanine according to General Procedure 1. 87% Yield. Foamy white solid. mp 60-61 °C.
[α]_{D}²⁵ = - 1.8 ° (c = 1.0, CHCl₃).
¹H NNR (CDCl₃) δ: 7.33-7.21 (m, 5H), 6.64 (brs, 1H), 5.13 (brd, 1H, *J* = 6.9 Hz), 4.32 (q, 1H, *J* = 7.0 Hz), 3.72 (s, 3H), 3.66 (s, 3H), 3.03 (d, 2H, *J* = 7.1 Hz), 2.48-2.38 (m, 2H), 2.08-1.84 (m, 3H), 1.60 (t, 1H, *J* = 2.9 Hz), 1.41 (s, 9H), 1.14-1.02 (m, 1H).
¹³C NMR (CDCl₃) δ: 172.5, 172.4, 171.3, 155.4, 136.8, 129.4, 128.5, 126.7, 80.1, 66.1, 55.3, 52.5, 51.7, 38.1, 34.1, 31.7, 28.2, 28.0, 26.3, 20.9.

### Example 4

### Synthesis of (1S,2S,5R,6S)-2-[(2'S)-(2'-tert-Butoxycarbonylamino)-3'-methylbutyryl]aminobicyclo[3.1.0]hexane-2,6-dicarboxylic acid dimethyl ester.

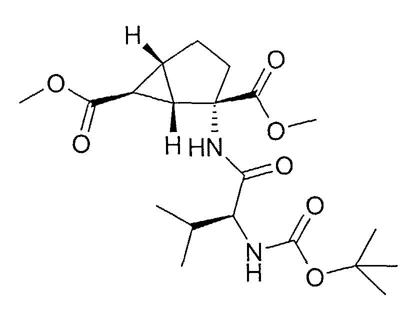

The title compound was prepared using commercially available N-BOC-(*L*)-valine according to General Procedure 1. 87% Yield. White foamy solid. mp 63-65 °C.
[α]_{D}²⁵ = - 1.12 ° (c = 1.16, CHCl₃).
¹H NMR (CDCl₃) δ: 6.75 (brs, 1H), 5.06 (brd, 1H, *J* = 8.8 Hz), 3.90 (dd, 1H, *J* = 8.9, 8.8 Hz), 3.73 (s, 3H), 3.66 (s, 3H), 2.54 (dd, 1H, *J* = 13.8, 8.3 Hz), 2.41 (dd, 1H, *J* = 5.9, 2.4 Hz), 2.23-1.88 (m, 4H), 1.71 (t, 1H, *J* = 2.9 Hz), 1.44 (s, 9H), 1.30-1.19 (m, 1H),0.97 (d, 3H, *J* = 6.8 Hz), 0.93 (d, 3H, *J* = 6.8 Hz).
¹³C NMR (CDCl₃) δ: 172.5, 172.4, 171.8, 155.8, 79.8, 66.2, 59.3, 52.4, 51.7, 34.3, 31.9, 30.9, 28.2, 27.9, 26.5, 21.1, 18.9, 17.6.

### Example 5

### Synthesis of (1S,2S,5R,6S)-2-[(2'S)-(2',6'-Bis-tert -butoxycarbonylamino)-hexanoyl]amino-bicyclo[3.1.0]hexane-2,6-dicarboxylic acid dimethyl ester

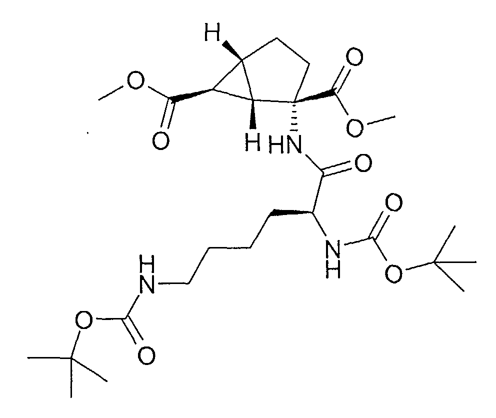

The title compound was prepared using commercially Nα-BOC-Nε-BOC-(*L*)-lysine according to General Procedure 1.
94% Yield. White foamy solid. mp 65-67 °C.
[α]_{D}²⁵ = -10.5° (c = 1.05, CHCl₃).
¹H NMR (CDCl₃) δ: 6.97 (brs, 1H), 5.12 (brd, 1H, *J* = 7.0 Hz), 4.76 (brs, 1H), 4.06 (brq, 1H, *J* = 7.4 Hz), 3.75 (s, 3H), 3.66 (s, 3H), 3.11 (AB system, 2H), 2.50 (dd, 1H, *J* = 13.8, 8.3 Hz), 2.42 (dd, 1H, *J* = 6.5, 2.7 Hz), 2.23-1.76 (m, 5H), 1.69 (t, 1H, *J* = 2.9 Hz), 1.63-1.11 (m, 5H), 1.43 (s, 9H), 1.42 (s, 9H).
¹³C NMR (CDCl₃) δ: 172.6, 172.5, 172.3, 156.1, 155.8, 80.1, 66.3, 53.7, 52.7, 51.9, 39.8, 34.4, 32.0, 31.7, 29.5, 28.4, 28.3, 28.1, 26.6, 22.3, 21.1.

### Example 6

### Synthesis of (1S,2S,5R,6S)-2-[(2'S)-(2'-tert-Butoxycarbonylamino)-3'-(3''-indolyl)-propionyl] aminobicyclo[3.1.0]hexane-2,6-dicarboxylic acid dimethyl ester

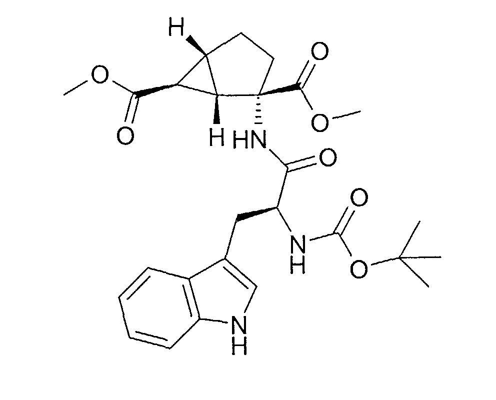

The title compound was prepared using commercially available N-BOC-*L*-tryptophan according to General Procedure 1.
90% Yield. White foamy solid. mp 89-91 °C.
[α]_{D}²⁵ = -12.7 ° (c = 1.15, CHCl₃).
¹H NNR (CDCl₃) δ: 8.36 (brs, 1H), 7.72 (brd, 1H, *J* = 7.0 Hz), 7.35 (dd, 1H, *J =* 7.2, 2.6 Hz), 7.16 (m, 2H), 7.06 (brd, 1H, *J =* 2.4 Hz), 6.24 (brs, 1H), 5.18 (brs, 1H), 4.44 (brdd, 1H, *J =* 8.1, 5.5 Hz), 3.70 (s, 3H), 3.64 (s, 3H), 3.29 (dd, 1H, *J* = 14.4, 5.5 Hz), 3.11 (dd, 1H, *J* = 14.4, 8.2 Hz), 2.41 (dd, 1H, *J* = 6.0, 2.6 Hz), 2.28 (dd, 1H, *J* = 13.5, 8.3 Hz), 2.10-1.94 (m, 2H), 1.82 (dd, 1H, *J* = 12.6, 7.6 Hz), 1.43 (brs, 1H, 9H), 1.43 (m, 1H), 0.99-0.83 (m, 1H).
¹³C NMR (CDCl₃) δ: 172.8, 172.4, 171.8, 155.5, 136.2, 127.2, 123.6, 122.2, 119.7, 118.9, 111.2, 110.6, 80.1, 66.1, 60.3, 54.6, 52.6, 51.8, 34.0, 31.9, 28.3, 28.1, 26.1, 21.0.

### Example 7

### Synthesis of (1S,2S,5R,6S)-2-[(2'S,3'S)-(2'-(2-tert-Butoxycarbonylamino-3-methyl-pentanoylamino)-bicyclo[3.1.0]hexane-2,6-dicarboxylic acid dimethyl ester

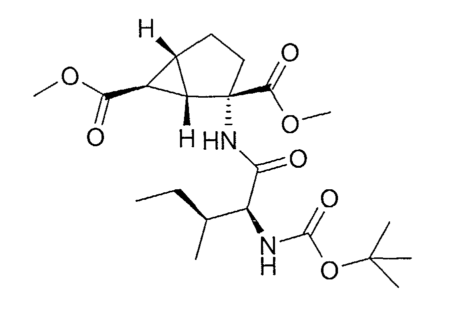

The title compound was prepared using commercially available N-BOC-(*L*)-isoleucine according to General Procedure 1.
79% Yield. White foamy solid.
¹H NMR (CDCl₃) δ: 6.75 (bs, 1 H); 5.06 (bd, *J* = 8.3 Hz, 1 H) ; 3.90 (dd, *J* = 8.9, 6.7 Hz, 1 H); 3.70 (s, 3H); 3.63 (s, 3 H); 2.54-2.39 (m, 2 H); 2.21-1.72 (m, 4 H); 1.67 (t, *J* = 3.2 Hz, 1 H); 1.60-1.40 (m, 1H); 1.41 (s, 9H); 1.22-1.0 (m, 2H); 0.91 (d, *J* = 6.7 Hz,3 H) ; 0.87 (t, *J* = 7.3 Hz, 3 H).

### Example 8

### Synthesis of (1S,2S,5R,6S)-2-[(2-tert-Butoxycarbonylaminoacetylamino)-bicyclo[3.1.0]hexane-2,6-dicarboxylic acid dimethyl ester

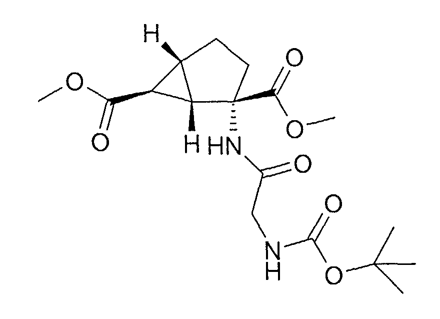

The title compound was prepared using commercially available N-BOC-glycine according to General Procedure 1.
88% Yield. White foamy solid.
¹H NMR (CDCl₃) δ: 7.01 (bs, 1 H); 5.28 (bs, 1 H); 3.75 (d, *J* = 5.6 Hz, 2 H); 3.72 (s, 3 H); 3.63 (s, 3 H); 2.54-2.38 (m, 2 H); 2.15-1.87 (m, 3 H); 1.68 (t, *J* = 2.9 Hz, 1 H); 1.43 (s, 9 H) ; 1.31-1.11 (m, 1 H).

### Example 9

### Synthesis of (1S,2S,5R,6S)-2-[(2'S)-(2'-tert-Butoxycarbonylamino)-(4'-methylthio)-butyryl]aminobicyclo[3.1.0]hexane-2,6-dicarboxylic acid dimethyl ester

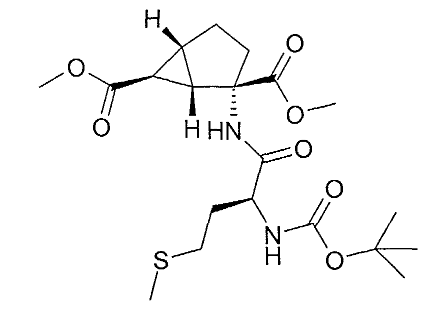

The title compound was prepared using commercially available N-BOC-(*L*)-methionine according to General Procedure 1.
89% yield. Foamy white solid
¹H NMR (CDCl₃) δ: 7.04 (brs, 1 H), 5.15 (d, 1 H, *J* = 8.3 Hz), 4.13 (m, 1 H), 3.61 (s, 3 H), 3.53 (s, 3 H), 2.48-2.25 (m, 4 H), 1.97 (s, 3 H), 2.15-1.71 (m, 5 H), 1.57 (t, 1 H, *J =* 3.0 Hz), 1.31 (s, 9 H), 1.13 (m, 1 H).
¹³C NMR (CDCl₃) δ: 172.4, 172.3, 171.5, 155.4, 79.9, 66.1, 52.7, 52.4, 51.6, 34.2, 31.8, 31.4, 29.7, 28.1 (3 C), 27.9, 26.4, 20.9, 15.0.

### Example 10

### Synthesis of (1S,2S,5R,6S)-2-[(2'S)-(2'-tert-Butoxycarbonylamino)-(4'-benzyloxycarbonyl)-butyryl]aminobicyclo[3.1.0]hexane-2,6-dicarboxylic acid dimethyl ester

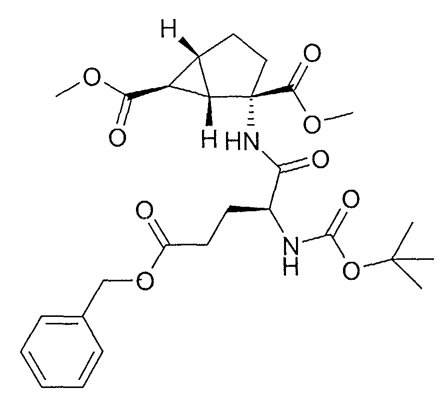

The title compound was prepared using commercially available N-BOC-(*L*)-γ-benzyl glutamic acid according to General Procedure 1.
67% yield. Foamy white solid.
¹H NMR (CDCl₃) δ: 7.32 (s, 5 H), 7.21 ( brs, 1 H), 5.28 (d, 1 H, *J* = 8.0 Hz), 5.10 (s, 2 H), 4.16 (m, 1 H), 3.69 (s, 3 H), 3.62 (s, 3 H), 2.58-2.43 (m, 3 H), 2.37 (dd, 1 H, *J* = 5.9, 2.1 Hz), 2.17-1.86 (m, 5 H), 1.68 (t, 1 H, *J* = 3.0 Hz), 1.41 (s, 9 H), 1.20 (m, 1 H) .
¹³C NMR (CDCl₃) δ: 173.2, 172.6, 172.5, 171.6, 155.6, 135.6, 128.5 (2 C), 128.2, 128.1, 79.9, 66.4, 66.2, 52.7, 52.6, 51.8, 34.4, 31.9, 30.0, 28.2 (3 C), 28.0, 27.9, 26.6, 21.0.

### Example 11

### Synthesis of (1S,2S,5R,6S)-2-[(2'S)-(2'-tert-Butoxycarbonylamino)-(3'-p-hydroxyphenyl)-propionyl]aminobicyclo[3.1.0]hexane-2,6-dicarboxylic acid dimethyl ester

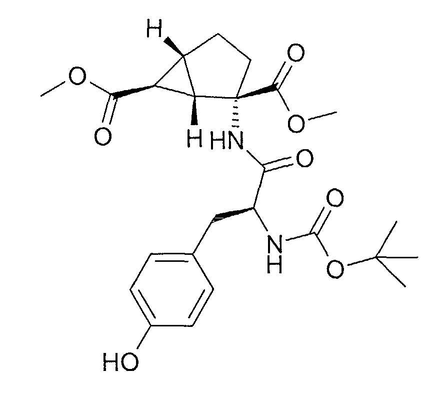

The title compound was prepared using commercially available N-BOC-(*L*)-tyrosine according to General Procedure 1.
73% yield. Foamy white solid.
¹H NMR (Methanol-*d*₄) δ: 7.03 (d, 2 H, *J* = 8.0 Hz), 6.72 (d, 2 H, *J* = 8.0 Hz), 5.21 (brs, 1 H), 4.28 (m, 1 H), 3.75 (s, 3 H), 3.63 (s, 3 H), 2.91 (m, 2 H), 2.39 (m, 2 H), 2.15-1.80 (m, 3 H), 1.59 (t, 1 H, *J* = 4.0 Hz), 1.39 (s, 9 H).
¹³C NMR (Methanol-*d*₄) δ: 173.6, 171.7, 155.3, 130.4 (2 C), 127.8, 121.2, 115.4 (2 C), 80.3, 66.2, 52.6, 51.8, 37.4, 34.1, 31.7, 28.1 (3 C), 26.3, 22.5, 21.0.

### Example 12

### Synthesis of (1S,2S,5R,6S)-2-[(2'S)-(2'-tert -Butoxycarbonylamino-3'-carbamoyl-propionylamino)]bicyclo[3.1.0]hexane-2,6-dicarboxylic acid dimethyl ester

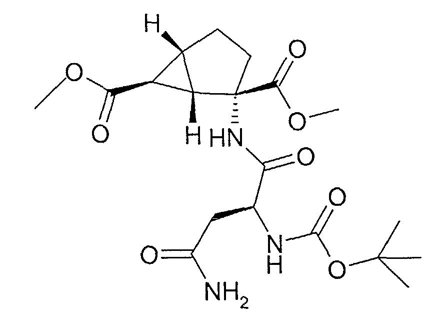

The title compound was prepared using commercially available N-BOC-(*L*)-asparagine according to General Procedure 1.
46% yield. Foamy white solid.
¹H NMR (CDCl₃) δ: 7.85 (brs, 1 H), 6.59 (brs, 1 H), 6.13 (d, 1 H, *J* = 7.8 Hz), 6.00 (brs, 1 H), 4.41 (m, 1 H), 3.67 (s, 3 H), 3.60 (s, 3 H), 2.75-2.35 (m, 4 H), 2.16-1.83 (m, 3 H), 1.15 (t, 1 H, *J* = 3.0 Hz), 1.43 (s, 9 H), 1.26 (m, 1 H).
¹³C NMR (CDCl₃) δ: 173.3, 172.7, 172.6, 171.9, 155.6, 80.0, 66.2, 52.5, 51.7, 50.8, 37.3, 34.1, 31.8, 28.6, 28.0, 26.3, 20.9.

### Example 13

### Synthesis of (1S,2S,5R,6S)-2-[2'R-tert-Butoxycarbonylamino)-propionyl]amino-bicyclo[3.1.0]hexane-2,6-dicarboxylic acid dimethyl ester.

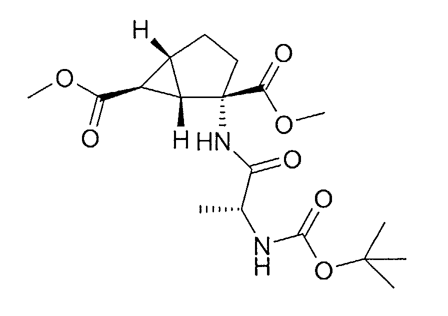

The title compound was prepared using commercially available N-BOC-(*D*)-alanine according to General Procedure 1..
93% Yield. White foam.
¹H NMR (CDCl3) δ: 7.03 (s, 1 H), 5.00 (s, 1 H), 4.12 (br m, 1 H), 3.72 (s, 3 H), 3.64 (s, 3 H), 2.52 (dd, J = 14.0, 8.5 Hz, 1 H), 2.39 (dd, J = 6.5, 3.0 Hz, 1 H), 2.18-2.10 (m, 1 H), 2.01 (app. quintet, J = 3.5 Hz, 1 H), 1.94 (dd, J = 13.5, 8.0 Hz, 1 H), 1.67 (t, J = 3.0 Hz, 1 H), 1.44 (s, 9 H), 1.31 (d, J = 7.0 Hz, 3 H), 1.24-1.17 (m, 1 H).
¹³C NMR (CDCl3) δ: 172.8, 172.7, 172.6, 155.7, 80.2, 66.1, 52.7, 51.8, 49.6, 34.4, 32.0, 28.2, 28.1, 26.6, 21.0, 17.4.

### Example 14

### Synthesis of (1S,2S,5R,6S)-2-[(2'S)-(1'-tert-Butoxycarbonylpyrrolidine-2'-carbonyl)amino]-bicyclo[3.1.0]hexane-2,6-dicarboxylic acid dimethyl ester

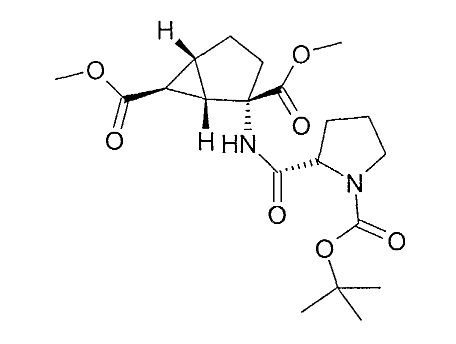

The title compound was prepared using commercially available N-BOC-(*L*)-proline according to General Procedure 1.
78% Yield. Foamy white solid.
¹H NNR (CDCl₃) δ: 4.2 (brs, 1H), 3.71 (s, 3H), 3.63 (s, 3H), 3.30 (brs, 2H), 2.47-2.45 (m, 2H), 2.03-1.77 (m, 8H), 1.65 (t, 1H, J = 3 Hz), 1.47 (s, 9H), 1.20-1.00 (m, 1H).

### Example 15

### Synthesis of (1S,2S,5R,6S)-2-[(2'S)-2-tert-butoxycarbonylamino-3-(tert-butyl-dimethyl-silanyloxy)-propionylamino]-bicyclo[3.1.0]hexane-2,6-dicarboxylic acid dimethyl ester.

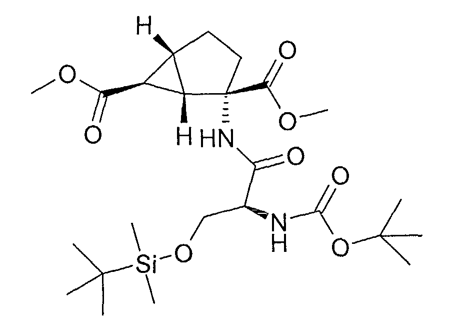

The title compound was prepared from (2*S*)-2-*tert*-butoxycarbonylamino-3-(*tert*-butyl-dimethyl-silanyloxy)-propionic acid methyl ester (which was prepared using a similar procedure described by Jacobson, et al, J. Med. Chem. 1999, 42(9), p. 1525-1536.) according to General Procedure 1.
82% yield. White foamy solid.
¹H-NMR (CDCl₃)δ: 7.44 (brs, 1 H); 5.38 (brs, 1 H); 4.16-4.05 (m, 1 H); 3.94 (dd, J = 9.7, 4.3 Hz, 1 H); 3.72 (s, 3 H); 3.65 (s, 3 H) ; 3.60-3.51 (m, 1 H); 2.64 (dd, J = 14.0, 8.6 Hz, 1 H); 2.30-2.11 (m, 2 H); 2.03-1.97 (m, 2 H); 1.72 (m, 1 H); 1.43 (s, 9 H); 1.26-1.09 (m, 1 H); 0.88 (s, 9 H); 0.11 (s, 3 H); 0.10 (s, 3 H).

### Example 16

### Synthesis of (1S,2S,5R,6S)-2-[(2'S,3'R)-2-tert-butoxycarbonylamino-3-(tert-butyl-dimethyl-silanyloxy)-butyrylamino]-bicyclo[3.1.0]hexane-2,6-dicarboxylic acid dimethyl ester.

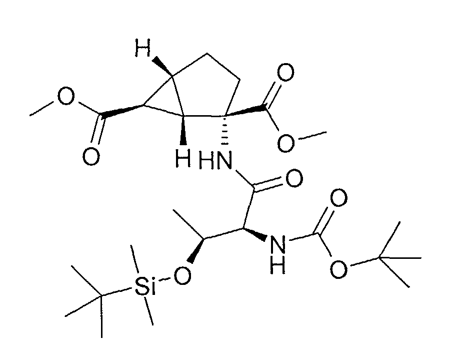

The title compound was prepared using (2*S*,3*S*) 2-tert-butoxycarbonylamino-3-(*tert*-butyl-dimethyl-silanyloxy)-butyric acid methyl ester (which was prepared using a similar procedure described by Jacobson, et al, J. Med. Chem. 1999, 42(9), p. 1525-1536) according to General Procedure 1.
70% yield. White foamy solid.
¹H-NMR (CDCl₃) : 7.55 (brs, 1 H); 5.52 (brd, J = 4.6 Hz, 1 H) ; 4.20-4.12 (m, 2 H) ; 3.73 (s, 3 H); 3.64 (s, 3 H); 2.64 (dd, J = 13.7, 8.1 Hz, 1 H); 2.31 (dd, J = 6.5, 2.7 Hz, 1 H); 2.26-2.10 (m, 1 H); 2.01-1.89 (m, 2 H); 1.71 (t, J = 1.6 Hz, 1 H) ; 1.41 (s, 9 H) ; 1.24-1.16 (m, 1 H); 1.11 (d, J = 6.4 Hz, 3 H); 0.89 (s, 9 H); 0.17 (s, 3 H); 0.13 (s, 3 H).

### Example 17

### Synthesis of (1S,2S,5R,6S) -2-[(2'S)-(2'-tert-Butoxycarbonylamino)-propionyl]amino-bicyclo[3.1.0]hexane-2,6-dicarboxylic acid

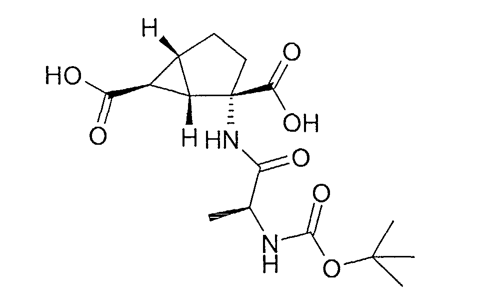

A solution of 2 M NaOH (5.45 L, 10.9 mol) was added to a solution of (1*S*,2*S*,5*R*,6*S*)-2-[(2'*S*) - (2'-*tert*-butoxycarbonylamino)-propionyl]amino-bicyclo[3.1.0]hexane-2,6-dicarboxylic acid dimethyl ester (4.52 mol, crude) in THF (2.8 L). The resulting mixture was stirred at ambient temperature for 3 h then extracted with CH₂Cl₂ (2 x 3 L). Ethyl acetate (5 L) and tetrahydrofuran (3 L) were then added to the aqueous phase. While stirring, concentrated HCl (970 mL) was added to the mixture until the pH = 2. The organic phase was dried (MgSO₄) and filtered. The aqueous phase was then extracted with ethyl acetate (5 L). The organic phase was dried (MgSO₄), filtered and combined with the previous organic phase. The combined organics were concentrated to a soft solid. Ethyl acetate was then added, and the mixture was concentrated to a soft solid. Ethyl acetate (3.5 L) was again added. The mixture was concentrated until a freely flowing suspension was present. Heptane ( 1.8 L) was then added, and the slurry was stirred at ambient temperature for 15 h. The solid was filtered, washed with heptane (3 L) then dried under vacuum to afford the title compound.
Yield 1.36 kg (84%) as an approximate 85:15 mixture of rotamers as a white solid
¹H NNR (DMSO-d₆) δ 12.20 (s, 2 H), 8.40 (s, 0.85 H), 8.36 (s, 0.15 H), 6.69 (d, *J* = 8.2 Hz, 0.85 H), 6.33 (br d, 0.15 H), 3.99 (quintet, *J* = 7.2 Hz, 0.85 H), 3.84 (br m, 0.15 H), 2.18-2.13 (m, 2 H), 1.91-1.84 (m, 1 H), 1.82-1.75 (m, 2 H), 1.46 (br s, 0.85 H), 1.43 (br s, 0.15 H), 1.35 (s, 9 H), 1.23-1.15 (m, 1 H), 1.13 (d, *J =* 6.9 Hz, 3 H).
¹³C NMR (methanol-d₄) δ 176.4, 176.0 (2 C), 157.5, 80.5, 67.3 (minor rotamer), 67.2 (major rotamer), 50.9, 35.6, 32.8, 29.3, 28.7, 27.4, 22.1, 18.5.
MS (EI) calcd for C₁₆H₂₈N₃O₇ (M + NH₄⁺) 374.20, found 374.24 *m*/*z*.

### Alternative Synthesis of (1S,2S,5R,6S)-2-[(2'S)-(2'-tert-Butoxycarbonylamino)-propionyl]amino-bicyclo[3.1.0]hexane-2,6-dicarboxylic acid

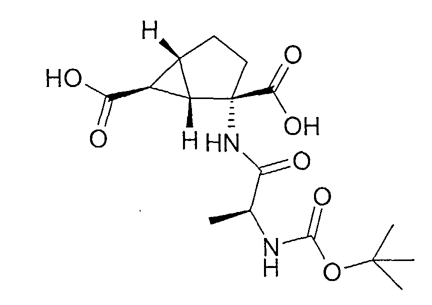

A solution of (1*S*,2*S*,5*R*,6*S*)-2-aminobicyclo[3.1.0]hexane-2,6-dicarboxylic acid monohydrate (85 g, 418 mmol) and MeOH (850 mL) was cooled to 10 °C. Thionyl chloride (199 g, 1.67 mol) was added at a rate such that the temperature did not exceed 20 °C. The solution was then heated to 50 °C and stirred for 6 h. Upon completion of the reaction, the solution was cooled to room temperature and concentrated to approximately 170 mL total volume under reduced pressure at 20 - 30 °C. Water (850 mL) was added, and the pH of the solution was adjusted to approximately pH 2.0 with 1.0 N NaOH (300 mL). The solution was concentrated under reduced pressure until the temperature reached approximately 40 °C. Methylene chloride (850 mL) was then added, and the pH of the solution was adjusted to pH 8 with 1.0 N NaOH (180 mL). The phases were separated, and the aqueous phase was extracted with CH₂Cl₂ (425 mL). The combined organic phases containing the corresponding dimethyl ester were concentrated to approximately 425 mL total volume and held for further processing.

In a separate reaction vessel a solution of *N*-t-butoxycarbonyl-L-alanine (83.2 g, 439 mmol) and 4-methylmorpholine (44.4 g, 439 mmol) in CH₂Cl₂ (712 mL) was cooled to -5 - -10 °C. Isobutyl chloroformate (59.9 g, 439 mmol) was then added at rate such that the temperature did not exceed -5 °C. Upon completion of the addition, the solution was stirred for 15 min. Simultaneously, CH₂Cl₂ (20 mL) was added to the dimethyl ester solution previously prepared, and this solution was cooled to -5 °C. The dimethyl ester solution (445 mL) was then added to the isobutyl mixed anhydride mixture. The cooling bath was removed, and the corresponding mixture was stirred for 30 min. A solution of 1.0 N HCl (445 mL) was then added. The phases were separated, and the organic phase was washed with 1.0 N HCl (445 mL). The organic phase was concentrated to approximately 180 mL total volume. THF (450 mL) was then added, and the resulting solution was concentrated to approximately 180 mL total volume. To this solution was added 1.0 N NaOH (1.67 L, 1.67 mol). The resulting mixture was heated to 40 °C, stirred for 1.5 h then cooled to room temperature. Ethyl acetate (2.4 L) was added, and the pH of the aqueous phase was adjusted to pH 2.1 with concentrated HCl (150 mL). The phases were separated, and the aqueous phase was extracted with ethyl acetate (800 mL). The combined organic phases were dried with MgSO₄, filtered and washed with EtOAc (2 x 320 mL). The resulting solution was then concentrated to approximately 400 mL total volume. Ethyl acetate (800 mL) was added, and the solution was concentrated to 400 mL). This ethyl acetate addition/concentration was repeated again, then heptane (640 mL) was added. The resulting mixture was stirred for 2 h, filtered and washed with a 2 : 1 mixture of heptane-ethyl acetate (2 x 320 mL) to afford 115.5 g (78% yield) of (1*S*,2*S*,5*R*,6*S*)-2-[(2'*S*)-(2'-*tert*-butoxycarbonylamino)-propionyl]amino-bicyclo[3.1.0] hexane-2,6-dicarboxylic acid as a white solid.

### Example 18

### Synthesis of (1S,2S,5R,6S) -2-[2'(S)-(2"(S)-tert-butoxycarbonylamino-propionylamino)-propionylamino]-bicyclo[3.1.0]hexane-2,6-dicarboxylic acid dimethyl ester.

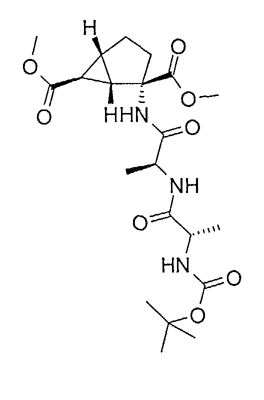

The title compound was prepared from 2' (S) - (2" (S) -tert-butoxycarbonylamino-propionylamino)-propionic acid according to General Procedure 1.
Yield 90%.
¹H NMR (CDCl₃): 7.26 (bs, 1 H); 6.66 (bd, J = 7.5 Hz, 1 H); 4.98 (bd, J = 7.0 Hz, 1 H); 4.46 (quint, J = 7.2 Hz, 1 H); 4.13 (m, J = 7.2 Hz, 1 H); 3.74 (s, 3H); 3.65 (s, 3H); 2.48-2.36 (m, 2H); 2.14-1.87 (m, 3H); 1.69 (t, J = 2.4 Hz, 1H); 1.43 (s, 9 H); 1.36 (d, J = 7.0 Hz, 3 H); 1.35 (d, J = 7.0 Hz, 3 H).

### Example 19

### Synthesis of (1S,2S,5R,6S)-2-[2'-(2"-tert-butoxycarbonylamino-4-methyl-pentanonylamino)-propionylamino]-bicyclo[3.1.0]hexane-2,6-dicarboxylic acid dimethyl ester.

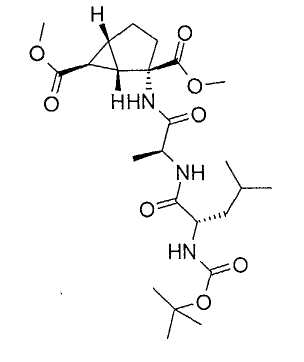

The title compound was prepared from 2(S)-(2'(S)-tert-butoxycarbonylamino-4-methyl-pentanonylamino)-propionic acid (preparation 3) according to General Procedure 1.
A mixture 2:1 of isomers was obtained.
Yield 95%.
¹H-NMR (CDCl₃): 7.09 (s, 1 H); 6.62 (d, J = 7.3 Hz, 1 H); 4.90 (d, J = 7.3 Hz, 1 H); 4.45 (m, 1 H); 4.08 (m, 1 H); 3.72 (s, 3 H); 3.65 (s, 3 H); 2.47-2.37 (m, 2 H); 2.07-1.53 (m, 7 H); 1.43 (s, 9 H); 1.34 (d, J = 7.0 Hz, 3 H); 1.40-1.20 (m, 1H); 0.95-0.92 (m, 6 H).

### Example 20

### Synthesis of (1S,2S,5R,6S)-2'S-[2"S-(2"'S-tert-Butoxycarbonylamino-propionylamino)-propionylamino]-propionic acid-bicyclo[3.1.0]hexane-2,6-dicarboxylic acid dimethyl ester.

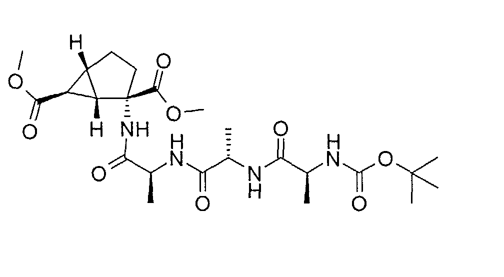

The title compound was prepared from 2S-[2'S-(2"S-tert-Butoxycarbonylamino-propionyl-amino)-propionylamino]-propionic acid (Preparation 4) according to General Procedure 1.

### Example 21

### Synthesis of (1S,2S,5R,6S)-2-[2'R-tert-Butoxycarbonylamino-4'-methyl)-pentanoyl]amino-bicyclo[3.1.0]hexane-2,6-dicarboxylic acid dimethyl ester.

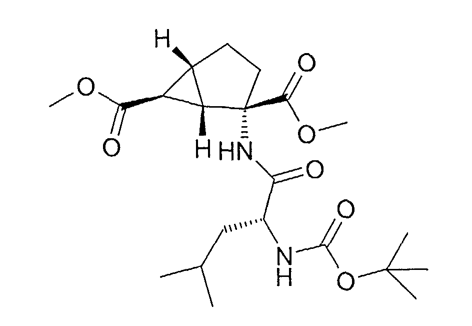

The title compound was prepared using commercially available N-BOC-(*D*)-leucine according to General Procedure 1.
¹H NMR (CD3OD): 7.92 (1H, br s), 4.57-4.48 (1H, m), 3.75 (3H, s), 3.70 (3H, s), 2.60 (1H, br s), 2.30-1.70 (8H, m), 1.43 (9H, s), 0.99-0.90 (6H, m)
MS: m/z 327 [M + H-CO2tBu]

### General Procedure 2

### General Procedure for 2'-tert-butoxycarbonylamino and 2,6-dimehtyl ester Deprotection. Hydrochloride Formation.

The corresponding 2'-N-BOC-2,6-dimethyl ester dipeptide derivative (1.0 equiv) was dissolved in THF and an equal volume of 2.5 N aqueous LiOH (10-20 equiv) was added. The reaction mixture was stirred at room temperature for 1-3 h. After dilution with water, the mixture was extracted twice with EtOAc and the organic extracts were discarded. The aqueous layer was acidified to pH = 1-2 with IN hydrochloric acid and thoroughly extracted with ethyl acetate (5 times). The combined organic extracts were washed with brine, dried over sodium sulfate, filtered and evaporated under vacuum to dryness. The crude N-BOC-dicarboxylic acid was dissolved in a saturated solution of hydrogen chloride gas in ethyl acetate (5-10 ml / mmol) and the mixture was stirred for 16 h. The resulting white precipitate was filtered, rinsed with ethyl ether and dried under high vacuum to afford the amino diacid hydrochloride salt as a fine white powder. If further purification was needed, the crude amino diacid was chromatogaphed over a C8 reverse phase support eluting with acetonitrile in water with 0.05% of trifluoroacetic acid to provide, after drying, the amino diacid as a zwitterion.

### Example 22

### Synthesis of (1S,2S,5R,6S)-2-[(2'S)-(2'-Amino)-propionyl]amino-bicyclo[3.1.0]hexane-2,6-dicarboxylic acid hydrochloride

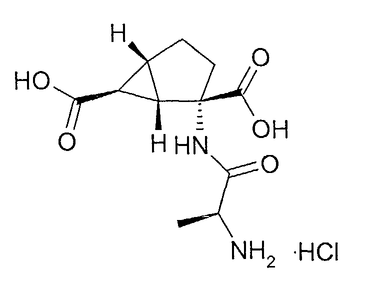

The title compound was prepared according to General Procedure 2.
80% Yield. White solid. mp >250 °C, dec.
[α]_{D}²⁵ = - 7.80 ° (c = 1.0, MeOH).
¹H NMR (Methanol-*d*₄) δ: 3.96 (q, 1H, *J =* 7.0 Hz), 2.47 (dd, 1H, *J* = 6.3, 2.7 Hz), 2.37 (dd, 1H, *J* = 13.6, 8.2 Hz), 2.18-1.92 (m, 3H), 1.66 (t, 1H, *J =* 3.0 Hz), 1.53 (d, 3H, *J =* 7.0 Hz), 1.46-1.34 (m, 1H).
¹³C NMR (Methanol-*d*₄) δ: 175.2, 174.7, 170.2, 66.4, 49.0, 36.6, 32.0, 28.5, 26.3, 21.2, 16.6.

### Example 23

### Synthesis of (1S,2S,5R,6S)-2-[(2'S)-(2'-Amino-4'-methyl)-pentanoyl]amino-bicyclo[3.1.0]hexane-2,6-dicarboxylic acid hydrochloride

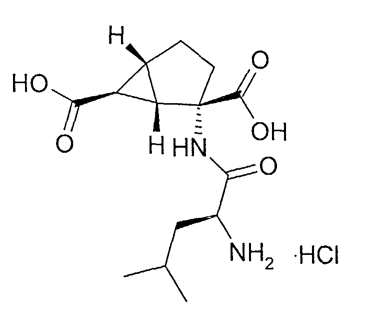

The title compound was prepared according to General Procedure 2.
48% Yield. White solid. mp 135-137 °C.
[α]_{D}²⁵ = + 0.3 ° (c = 1.0, MeOH).
¹H NMR (Methanol-*d*₄) δ: 3.84 (brt, 1H, *J* = 6.6 Hz), 2.52 (dd, 1H, *J* = 6.2, 2.8 Hz), 2.31 (dd, 1H, *J* = 13.5, 8.6 Hz), 2.21-1.89 (m, 3H), 1.74-1.59 (m, 4H), 1.44 (m, 1H), 1.02 (d, 3H, *J* = 6.4 Hz), 0.99 (d, 3H, *J* = 6.2 Hz).
¹³C NMR (Methanol-*d*₄) δ: 175.3, 174.9, 169.7, 66.6, 51.8, 40.6, 34.4, 32.0, 28.7, 26.1, 24.4, 21.8, 21.5, 21.3.

### Example 24

### Synthesis of (1S,2S,5R,6S)-2-[(2'S)-(2'-Amino)-3'-phenylpropionyl]amino-bicyclo[3.1.0]hexane-2,6-dicarboxylic acid hydrochloride.

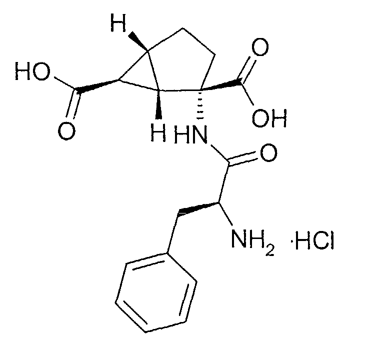

The title compound was prepared according to General Procedure 2.
44% Yield. White solid. mp 122-123 °C.
[α]_{D}²⁵ = - 2.10 ° (c = 1.0, MeOH).
¹H NMR (Methanol-*d*₄) δ: 7.41-7.26 (m, 5H), 4.13 (dd, 1H, *J* = 7.6, 6.1 Hz), 3.28 (dd, 1H, *J* = 14.2, 6.2 Hz), 3.07 (dd, 1H, *J* = 14.2, 7.6 Hz), 2.55 (dd, 1H, *J =* 6.4, 2.8 Hz), 2.31-1.89 (m, 4H), 1.64 (t, 1H, *J* = 2.8 Hz), 1.35 (m, 1H).
¹³C NMR (Methanol-*d*_{*4*}) δ: 178.8, 178.3, 172.2, 137.9, 133.2, 132.6, 131.3, 70.0, 57.9, 41.0, 37.7, 35.4, 32.3, 29.4, 24.7.

### Example 25

### Synthesis of (1S,2S,5R,6S)-2-[(2'S)-(2'-Amino)-3'-methylbutyryl]amino-bicycle[3.1.0]hexane-2,6-dicarboxylic acid hydrochloride.

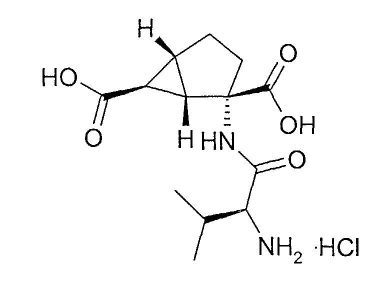

The title compound was prepared according to General Procedure 2.
64% Yield. White solid. mp 217-219 °C.
[α]_{D}²⁵ = + 5.93 ° (c = 0.86, MeOH).
¹H NMR (D₂O) δ: 3.78 (d, 1H, *J* = 5.8 Hz), 2.56 (dd, 1H, *J* = 6.9, 2.9 Hz), 2.19-1.95 (m, 5H), 1.66 (t, 1H, *J* = 3.0 Hz), 1.50 (m, 1H), 0.99 (d, 3H, *J* = 6.9 Hz), 0.98 (d, 3H, *J* = 6.9 Hz).
¹³C NMR (D₂O + Methanol-*d*_{*4*}) δ: 177.0, 176.0, 169.3, 66.3, 58.1, 34.0, 31.3, 30.0, 29.5, 25.1, 20.9, 17.3, 16.9.

### Example 26

### Synthesis of (1S,2S,5R,6S)-2-[(2'S)-(2',6'-Diamino)-hexanoyl]amino-bicyclo[3.1.0]hexane-2,6-dicarboxylic acid dihydrochloride.

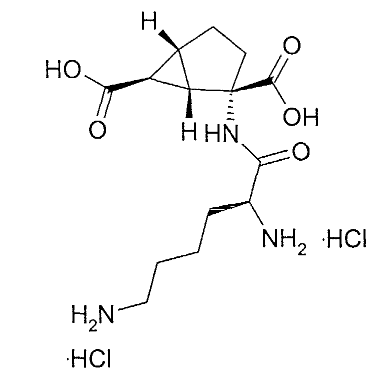

The title compound was prepared according to General Procedure 2.
82% Yield. White solid. mp 75-77 °C.
[α]_{D}²⁵ = - 1.10 ° (c = 1.0, MeOH).
¹H NMR (D₂O) δ: 3.90 (t, 1H, *J* = 6.3 Hz), 2.84 (brt, 2H, *J* = 7.6 Hz), 4.42 (dd, 1H, *J* = 6.3, 2.8 Hz), 2.09-1.73 (m, 6H), 1.59-1.26 (m, 6H).
¹³C NMR (D₂O + Methanol-d₄) δ: 177.1, 176.3, 169.4, 66.1, 52.4, 38.8, 33.8, 31.2, 30.2, 29.3, 26.3, 25.1, 20.8, 20.6.

### Example 27

### Synthesis of (1S,2S,5R,6S)-2-[(2'S)-(2'-Amino)-3'-(3''-indolyl)-propionyl]amino-bicyclo[3.1.0]hexane-2,6-dicarboxylic acid.

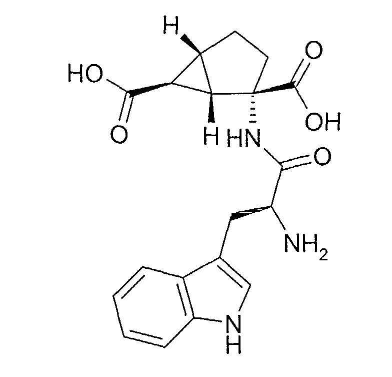

The title compound was prepared according to General Procedure 2.
41% Yield. White solid. mp 96-98 °C.
[α]_{D}²⁵ = + 1.35° (c = 0.74, MeOH).
¹H NMR (D₂O) δ: 7.51 (d, 1H, *J* = 7.8 Hz), 7.38 (d, 1H, *J* = 7.9 Hz), 7.17-7.00 (m, 2H), 7.08 (brs, 1H), 4.18 (t, 1H, *J* = 7.1 Hz), 3.24 (AB system, 2H), 2.37 (dd, 1H, *J* = 6.5, 2.8 Hz), 2.09-1.65 (m, 4H), 1.34 (t, 1H, *J* = 2.9 Hz), 1.22-1.12 (m, 1H).
¹³C NMR (D₂O + Methanol-d₄) δ: 177.9, 176.8, 170.6, 137.2, 127.3, 126.2, 123.2, 120.5, 119.0, 113.1, 107.1, 67.0, 54.0, 34.3, 32.1, 30.4, 27.9, 25.8, 21.4.

### Example 28

### Synthesis of (1S,2S,5R,6S)-2-[(2'S,3'S)-(2'-Amino-3'-methylpentanoylamino)-bicyclo[3.1.0]hexane-2,6-dicarboxylic acid hydrochloride.

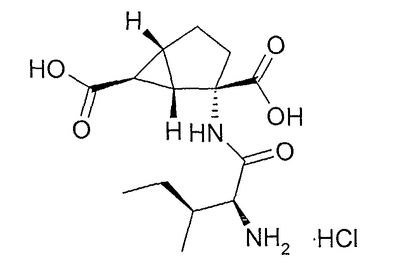

The title compound was prepared according to General Procedure 2.
64% Yield. White solid. mp>300°C.
[α]_{D}²⁵ = -5° (c=1,1N HCl).
¹H NNR (MeOH-d₄) δ: 8.93 (s, 1 H); 3.73 (d, *J* = 5.4 Hz, 1 H); 2.54 (dd, *J* = 6.4, 3.0 Hz, 1 H); 2.35-1.91 (m, 5 H); 1.67-1.15 (m, 4 H); 1.06 (d, *J* = 7.0 Hz, 3 H); 0.98 (t, *J* = 7.3 Hz, 3 H) .
¹³C NMR (MeOH-d₄) δ: 176.2, 175.7, 169.6, 67.4, 58.7, 38.1, 35.3, 32.9, 29.8, 26.9, 25.2, 22.2, 15.1, 11.7.

### Example 29

### Synthesis of (1S,2S,5R,6S)-2-(2-Amino-acetylamino)-bicyclo[3.1.0]hexane-2,6-dicarboxylic acid.

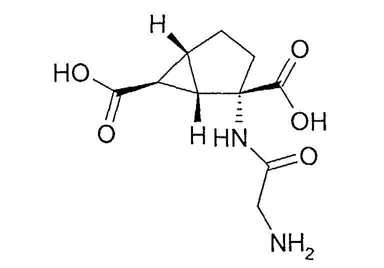

The title compound was prepared according to General Procedure 2.
(44% yield) White solid. mp: 149-156°C.
¹H NNK (MeOH-d₄) δ: 3.68 (s, 2 H); 2.37 (dd, *J* = 6.2, 3.0 Hz, 1 H); 2.19-2.08 (m, 1 H); 2.01-1.85 (m, 3 H) ; 1.59 (t, *J* = 2.7 Hz, 1 H); 1.37-1.21 (m, 1 H).

### Example 30

### Synthesis of (1S,2S,5R,6S)-2-[(2'S)-(2'-Amino)-(4'-methylthio)-butyryl]amino-bicyclo[3.1.0]hexane-2,6-dicarboxylic acid.

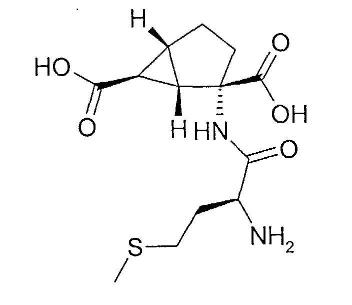

The title compound was prepared according to General Procedure 2.
(78% Yield) White solid. mp: 164 °C.
[α]_{D}²⁵ = + 13.4 ° (c = 1.1, MeOH).
¹H NMR (Methanol-*d*₄) δ: 3.97 (t, 1 H, *J* = 6.4 Hz), 2.64-2.56 (m, 2 H), 2.48-2.38 (m, 2 H), 2.11 (s, 3 H), 2.19-1.91 (m, 5 H), 1.66 (t, 1 H, *J* = 3.0 Hz), 1.38 (m, 1 H).
¹³C NMR (Methanol-*d*₄) δ: 174.7, 174.1, 168.3, 66.1, 52.0, 34.1, 31.5, 30.8, 28.1, 28.0, 25.9, 20.8, 13.6.
MS (Electrospray): 317 (M⁺+1), 229.

### Example 31

### Synthesis of (1S,2S,5R,6S)-2-[(2'S)-(2'-amino)-(4'-carboxy)-butyryl]amino-bicyclo[3.1.0]hexane-2,6-dicarboxylic acid hydrochloride.

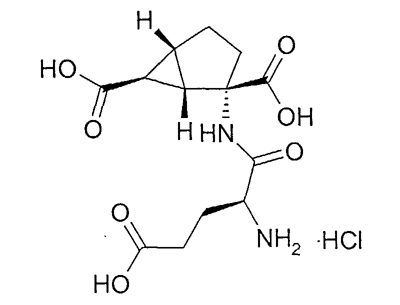

The title compound was further purified by solid phase (C18) extraction eluting with methanol in water according to General Procedure 2.
84% Yield.White solid mp: 193 °C.
[α]_{D}²⁵ = + 19.1 ° (c = 0.90, MeOH).
¹H NMR (Methanol-*d*₄) δ: 3.92 (t, 1 H, *J* = 6.1 Hz), 2.59-2.50 (m, 2 H), 2.42 (dd, 2 H, *J* = 6.5, 2.8 Hz), 2.19-1.92 (m, 5 H), 1.68 (t, 1 H, *J* = 3.0 Hz), 1.37 (m, 1H).
¹³C NMR (Methanol-*d*₄) δ: 174.9, 174.6, 174.1, 168.4, 66.1, 52.0, 34.2, 31.5, 28.6, 28.0, 26.2, 26.1, 20.9.
MS (Electrospray): 315 (M⁺+1), 297, 279.

### Example 32

### Synthesis of (1S,2S,5R,6S)-2-[(2'S)-(2'-amino)-(3'-p-hydroxyphenyl)-propionyl]amino-bicyclo[3.1.0]hexane-2,6-dicarboxylic acid.

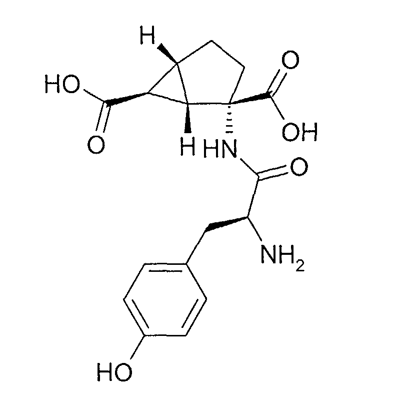

The title compound was prepared according to General Procedure 2.
35 % Yield. White solid mp: 169 °C.
[α]_{D}²⁵ = -2.4 ° (c = 0.95, MeOH).
¹H NMR (Methanol-*d*₄) δ: 7.13 (d, 2 H, *J* = 8.6 Hz), 6.79 (d, 2 H, *J* = 8.6 Hz), 4.02 (dd, 1 H, *J* = 7.5, 5.9 Hz), 3.18 (dd, 1 H, *J* = 14.2, 5.9 Hz), 2.94 (dd, 1 H, *J* = 14.4, 7.5 Hz), 2.54 (dd, 1 H, J = 6.3, 2.8 Hz), 2.34-1.89 ( m, 4 H), 1.66 (t, 1 H, J = 3.0 Hz), 1.35 (m, 1 H).
¹³C NMR (Methanol-*d*_{*4*}) δ: 174.9, 174.3, 168.5, 156.7, 130.3, 124.5, 115.4, 66.0, 54.2, 36.3, 33.9, 31.5, 28.3, 25.5, 20.8.
MS (Electrospray): 349 (M⁺+1), 331.

### Example 33

### Synthesis of (1S,2S,5R,6S)-2-[(2'R)-(2'-Amino)-propionyl]amino-bicyclo[3.1.0]hexane-2,6-dicarboxylic acid hydrochloride.

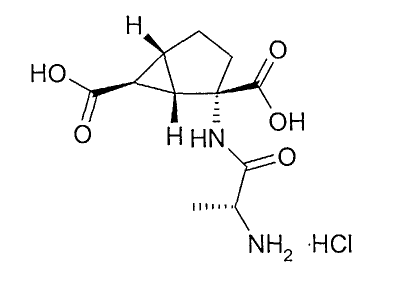

The title compound was prepared according to General Procedure 2.
¹H NMR (Methanol-*d*₄) δ: 3.92 (q, *J* = 7.1 Hz, 1 H), 2.56 (dd, *J* = 6.4, 2.7 Hz, 1 H), 2.25 (*J* =14.0, 8.5 Hz, 1 H), 2.13-2.06 (m, 1 H), 2.01 (m, 1 H), 1.95 (dd, *J* = 12.8, 8.0 Hz, 1 H), 1.62 (t, J = 3.0 Hz, 1 H), 1.51 (d, J = 6.9 Hz, 3 H), 1.43-1.37 (m, 1H).
¹³C NMR (Methanol-*d*₄) δ: 176.4, 175.7, 171.2, 67.3, 50.0, 35.5, 32.8, 30.0, 26.7, 21.9, 17.7.

### Example 34

### Synthesis of (1S,2S,5R,6S)-2-[(2'S)-(Pyrrolidine-2-carbonyl)] amino-bicyclo[3.1.0]hexane-2,6-dicarboxylic acid hydrochloride.

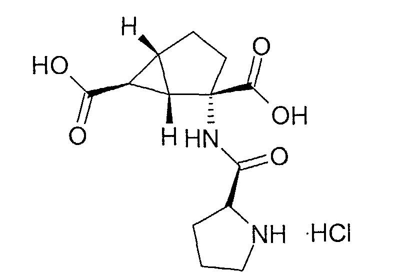

The title compound was prepared according to General Procedure 2.
90% Yield. White solid.
¹H NMR (Methanol-*d*₄) δ: 4.31-4.24 (m, 1H), 3.37-3.28 (m, 2H), 2.45-2.41 (m, 3H), 2.14-1.98 (m, 6H), 1.65 (t, 1H, *J* = 2Hz), 1.44-1.32 (m, 1H) ppm.
¹³C NMR (Methanol-d₄) δ: 174.7, 174.1, 168.4, 66.2, 59.5, 46.1, 34.3, 31.6, 29.6, 28.0, 26.0, 23.4, 20.8 ppm.

### Example 35

### Synthesis of (1S,2S,5R,6S)-2-[2'(S)-(2"(S)-Aminopropionylamino)-propionylamino]-bicyclo[3.1.0]hexane-2,6-dicarboxylic acid.

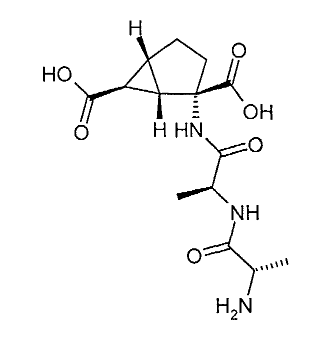

The title compound was prepared according to general procedure 2. Purification by reverse phase chromatography provided a white solid.
45% Yield. mp = 193°C.
[α]_{D}²⁵ = -25° (c=1.05, MeOH).
I.R. (KBr) : 3380, 3285, 1664 cm⁻¹.
¹H NMR (MeOH-d₄) δ: 8.67 (bs, 1 H) ; 4.43 (q, *J* = 7.3 Hz, 1 H); 3.93 (q, *J* = 7.0 Hz, 1 H); 2.45-2.34 (m, 2 H); 2.16-1.88 (m, 3 H); 1.66 (t, *J* = 3.2 Hz, 1 H); 1.50 (d, *J* = 7.3 Hz, 3 H); 1.35 (d, *J* = 7.3 Hz, 3 H); 1.33 (m, 1 H).
¹³C NMR (MeOH-d₄) δ: 176.8, 176.3, 175.0, 171.0, 67.7, 50.6, 50.4, 36.1, 33.3, 29.8, 27.8, 22.7, 18.6, 18.0.

### Example 36

### Synthesis of (1S,2S,5R,6S)-2-[2'S-(2"S-amino-4-methylpentanonylamino)-propionylamino]-bicyclo[3.1.0]hexane-2,6-dicarboxylic acid.

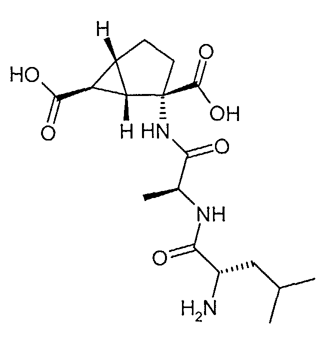

The title compound was prepared according to general procedure 2 with final purification by reverse phase chromatography.
30% Yield of a white solid. mp = 195-201°C
[α]D₂₅ = -10° (c=1.01, MeOH).
I.R. (KBr): 3414, 1669 cm⁻¹.
¹H NMR (MeOH-d₄) δ: 4.46 (q, J = 7.0 Hz, 1 H); 3.87 (m, 1 H); 2.45-2.35 (m, 2 H); 2.16-1.60 (m, 7 H); 1.37 (d, J = 7.3 Hz, 3 H); 1.39-1.20 (m, 1H); 0.99 (m, 6 H).
¹³C NMR (DMSO-d₆) δ: 174.9, 174.7, 172.8, 169.3, 66.2, 51.8, 48.8, 41.0, 34.8, 32.5, 28.3, 27.0, 24.6, 23.7, 23.2, 21.8, 19.6.

### Example 37

### Synthesis of (1S,2S,5R,6S)-2-{2S-[2S-(2S-Aminopropionylamino)-propionylamino]-propionylamino}bicyclo[3.1.0]hexane-2,6-dicarboxylic acid hydrochloride.

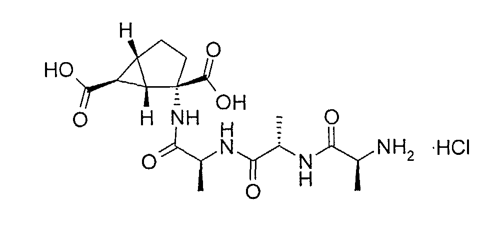

The title compound was prepared according to general procedure 2. A white solid was obtained.
¹H NMR (D₂O) : 4.19-4.13 (2H, m), 3.97-3.90 (1H, m), 2.33 (1H, dd, 8.3 Hz, 2.3 Hz), 2.14-2.09 (1H, m), 1.95-1.83 (2H, m), 1.59-1.55 (1H, m), 1.39-1.10 (11H, m)
MS: m/z 399 [M + H]

### Example 38

### Synthesis of (1S,2S,5R,6S)-2-[(2'S)2'-Amino-3'-hydroxypropionylamino)-bicyclo[3.1.0]hexane-2,6-dicarboxylic acid.

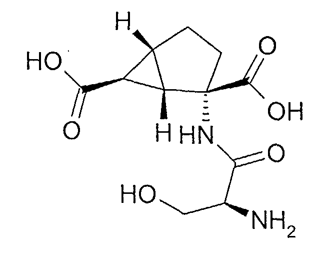

(1S,2S,5R,6S)-Methyl 2-[(2'S)-2-tert-butoxycarbonylamino-3-(tert-butyl-dimethyl-silanyloxy)-propionylamino]-bicyclo[3.1.0]hexane-2,6-dicarboxylate was dissolved in dry THF (10 mL) under nitrogen and 1M tetrabutylammonium fluoride in THF was added. The reaction was stirred at rt for 1h, the solvent was evaporated and the residue was purified by silica gel chromatography eluting with ethyl acetate. The desilyated material was subjected to the general procedure for 2'-*tert*-butoxycarbonylamino and 2,6-dimehtyl ester deprotection. The title compound was purified by HPLC.
11% yield. Very hygroscopic white solid.
¹H-NMR (CDCl₃) δ: 4.01-3.71 (m, 3 H); 2.39 (dd, J = 6.4, 3.0 Hz, 1 H); 2.19-1.86 (m, 4 H); 1.59 (t, J = 2.7 Hz, 1 H); 1.41-1.25 (m, 1 H).
¹³C-NMR (D₂O) δ: 177.0, 175.9, 167.8, 66.4, 60.2, 54.4, 34.2, 31.4, 29.1, 25.5, 20.7.
[α]_{D}²⁵ = -14° [c=1, 1N HCl].

### Example 39

### Synthesis of (1S,2S,5R,6S)-2-[(2'S,3'R)-2'-amino-3'-hydroxy)-butyryl)amino-bicyclo[3.1.0]hexane-2,6-dicarboxylic acid.

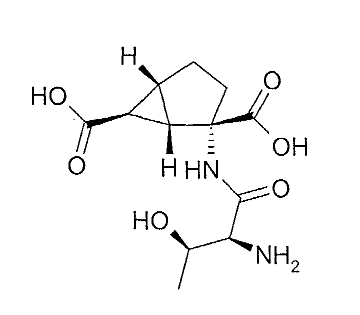

(1S,2S,5R,6S)-Nethyl 2*-*[(2'S,3'R)-2-tert-butoxycarbonylamino-3-(*tert*-butyl-dimethyl-silanyloxy)-butyrylamino]-bicyclo[3.1.0]hexane-2,6-dicarboxylate was dissolved in dry THF (10 mL) under nitrogen and 1M tetrabutylammonium fluoride was added. The reaction was stirred at rt for 1h, the solvent was evaporated and the residue was purified by silica gel chromatography eluting with ethyl acetate. The desilylated material was subjected to the general procedure for 2'-*tert-*butoxycarbonylamino and 2,6-dimehtyl ester deprotection. The title compound was purified by HPLC.
14% yield. Hygroscopic white solid.
[□]_{D}²⁵ = -13° [c=1, 1N HCl].
¹H-NNR (D₂O)δ: 4.03 (dq, J = 7.3, 6.4 Hz, 1 H); 3.77 (d, J = 7.3 Hz, 1 H); 2.52 (dd, J = 6.2, 2.7 Hz, 1 H); 2.27-2.07 (m, 4 H); 1.69 (t, J = 3.0 Hz, 1 H); 1.55-1.39 (m, 1 H); 1.28 (d, J = 6.5 Hz, 3 H).
¹³C-NMR (D₂O)δ: 176.9, 175.9, 167.9, 66.4, 66.3, 58.6, 33.8, 31.3, 29.2, 25.2, 20.8, 18.7.

### Example 40

### Synthesis of (1S,2S,5R,6S)-2-[(2'S)-(2'-Amino)-propionyl]amino-bicyclo[3.1.0]hexane-2,6-dicarboxylic acid methane sulfonate.

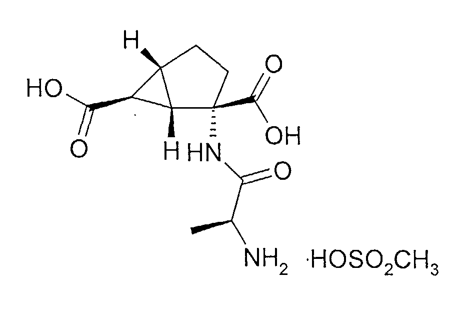

A solution of (1S,2S,5R,6S)-2-[(2'S)-(2'-tert-butoxycarbonylamino)-propionyl]amino-bicyclo[3.1.0]hexane-2,6-dicarboxylic acid (1.07 g, 3.00 mmol), methanesulfonic acid (584 ul, 9.00 mmol) and dioxane (10 mL) was stirred for 48 h. The mixture was filtered and dried to afford (1S, 2S, 5R, 6S)-2-[(2'S)- (2'-Amino)- propionyl]aminobicyclo[3.1.0]hexane-2,6-dicarboxylic acid methane sulfonate as a crude, white, amorphous solid (1.05 g). A sample of this solid (1.0 g) was dissolved in MeOH (10 mL). The solution was concentrated to 3.3 g total weight and seed crystals were added. Ethyl acetate (10 mL) was then added to the mixture over a period of 15 min. The mixture was stirred for 30 min, filtered and dried under vacuum to afford 830 mg of the title compound as a white, crystalline solid.
Yield 78%
¹H NMR (methanol-d₄) δ 3.96 (q, J = 7.1 Hz, 1 H), 2.71 (s, 3 H), 2.45 (dd, J = 6.4, 2.7 Hz, 1 H), 2.38 (dd, J = 13.9, 8.4 Hz, 1 H), 2.20-2.08 (m, 1 H), 2.01-1.93 (m, 2 H), 1.67 (t, J = 2.9 Hz, 1 H), 1.52 (d, J = 7.0 Hz, 3 H), 1.46-1.35 (m, 1 H)
¹³C NMR (methanol-d₄) δ 176.3, 175.7, 171.2, 67.4, 50.0, 39.5, 35.7, 33.1, 29.5, 27.4, 22.2, 17.6.
Anal. Calcd for C₁₂H0N₂O₈S: C, 40.90; H, 5.72; N, 7.95.
Found: C, 40.81; H, 5.69; N, 7.83.

### Example 41

### Synthesis of (1S,2S,5R,6S)-2-[(2'S)-(2'-Amino)-propionyl]amino-bicycle[3.1.0]hexane-2,6-dicarboxylic acid.

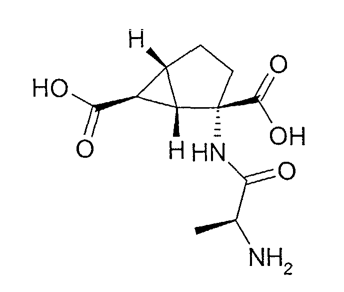

(1S, 2S, 5R, 6S)-2-[(2'S)- (2'-Amino)- propionyl]aminobicyclo[3.1.0]hexane-2,6-dicarboxylic acid hydrochloride (1.0 g, 3.42 mmol) was dissolved in water (1 mL), and 1.0 N NaOH (3.42 mL, 3.42 mmol) was added. The solution was maintained in the refrigerator for 24 h. The solution remained clear. Acetone (2 mL) was added, and the solution was stored in the refrigerator for 16 h. A white solid precipitated out of solution, and mixture could not be stirred. Acetone (4 mL) was added, and the mixture was stirred at rt, then filtered and dried to afford 630 mg of the title compound as a white crystalline solid which contained 2-4% NaCl.
Yield 72%
¹H NMR (methanol-d₄) δ 3.93 (q, J = 7.1 Hz, 1 H), 2.48 (dd, , J = 6.6, 2.9 Hz, 1 H), 2.32 (dd, , J = 13.5, 8.4 Hz, 1 H), 2.20-2.08 (m, 1 H), 2.01-1.90 (m, 2 H), 1.61 (t, , J = 2.9 Hz, 1 H), 1.51 (d, , J = 7.0 Hz, 3 H), 1.48-1.33 (m, 1 H)
¹³C NMR (methanol-d₄) δ 176.9 (2 C), 171.1, 68.0, 50.1, 35.9, 33.2, 29.7, 27.3, 22.5, 17.6.

### Example 42

### Synthesis of (1S,2S,5R,6S)-2-[2'R-Amino-4'-methyl)-pentanoyl]amino-bicyclo[3.1.0]hexane-2,6-dicarboxylic acid hydrochloride.

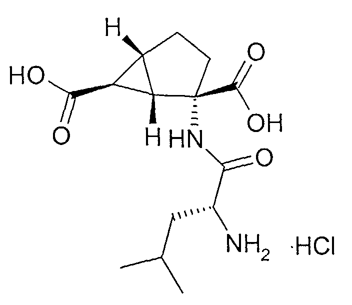

The title compound was prepared according to General Procedure 2.
¹H NMR (CD₃OD): 3.62 (1H, t, 7.2 Hz), 3.02 (1H, t), 2.18 (1H, dd, 2.3 Hz, 6.4 Hz), 2.04-1.91 (1H, m), 1.90-0.80 (7H, m), 0.68 (6H, 2 x t, 6.7 Hz, 6.8 Hz)
MS: m/z 299 [M + H]

## Claims

1. A compound of the formula I 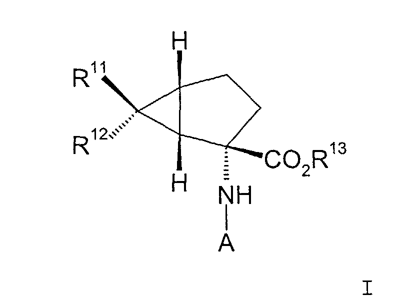 wherein
R¹¹ is CO₂R¹⁴ and R¹² is hydrogen or fluoro; or R¹¹ is hydrogen or fluoro and R¹² is CO₂R¹⁴;
R¹³ and R¹⁴ are, independently, hydrogen, (1-10C) alkyl, (2-4C) alkenyl, aryl or arylalkyl;
A is (Q)ₚ- ;
p is any integer from 1-10; and
Q is independently selected, each time taken, from the group amino acyl;
provided that the compound is not one in which R¹¹ is CO₂R¹⁴; R¹², R¹³ and R¹⁴ are hydrogen; p is 1; and Q is L-alanyl;
or a pharmaceutically acceptable salt thereof.

2. A compound of the formula I 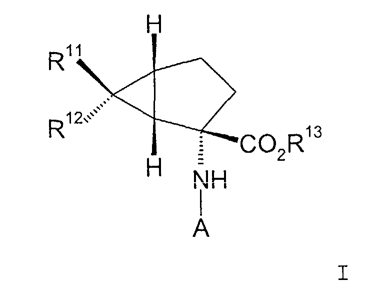 wherein
R¹¹ is CO₂R¹⁴ and R¹² is hydrogen or fluoro; or R¹¹ is hydrogen or fluoro and R¹² is CO₂R¹⁴;
R¹³ and R¹⁴ are, independently, hydrogen,
(1-10C) alkyl, (2-4C) alkenyl, aryl or arylalkyl;
A is (Q)ₚ- ;
p is any integer from 1-10; and
Q is independently selected, each time taken, from the group amino acyl wherein amino acyl is an α-amino acid;
provided that the compound is not one in which R¹¹ is CO₂R¹⁴; R¹², R¹³ and R¹⁴ are hydrogen; p is 1; and Q is L-alanyl;
or a pharmaceutically acceptable salt thereof.

3. The compound (or salt thereof) of Claims 1 or 2 wherein (1-10C) alkyl is methyl.

4. The compound (or salt thereof) of Claims 1 or 2 wherein
R¹¹ is CO₂R¹⁴;
R¹², R¹³, R¹⁴ and R¹⁵ are hydrogen; and
p is any integer from 1-3.

5. The compound (or salt thereof) of Claims 1, 2, or 4 wherein Q is independently selected, each time taken, from L-alanyl, glycyl, L-leucyl, L-phenylalanyl, L-valyl, L-lysyl, L-tryptophyl, L-isoleucyl, L-methionyl, L-glutamyl, L-tyrosyl, D-alanyl, L-prolyl, L-serinyl, D-leucyl, L-asparagyl and L-threonyl.

6. The compound of Claims 1, 2, 4, or 5 which is selected from:
a) (1*S*,2*S*,5*R*,6*S*)-2-[(2'*S*)-(2'-Amino)-3'-phenylpropionyl]amino-bicyclo[3.1.0]hexane-2,6-dicarboxylic acid hydrochloride;
b) (1*S*,2*S*,5*R*,6*S*)-2-[(2'*S*)-(2'-Amino)-3'-methylbutyryl]amino-bicyclo[3.1.0]hexane-2,6-dicarboxylic acid hydrochloride;
c) (1*S*,2*S*,5*R*,6*S*)-2-[(2'*S*,3'*S*)-(2'-Amino-3'-methylpentanoylamino)-bicyclo[3.1.0]hexane-2,6-dicarboxylic acid hydrochloride;
d) (1*S*,2*S*,5*R*,6*S*)-2-(2-Amino-acetylamino)-bicyclo[3.1.0]hexane-2,6-dicarboxylic acid;
e) (1*S*,2*S*,5*R*,6*S*)-2-[(2'*S*)-(2'-Amino)-(4'-methylthio)-butyryl]amino-bicyclo[3.1.0]hexane-2,6-dicarboxylic acid;
f) (1*S*,2*S*,5*R*,6*S*)-2-[(2'*S*)-(2'-amino)-(3'-*p*-hydroxyphenyl)-propionyl]amino-bicyclo[3.1.0]hexane-2,6-dicarboxylic acid;
g) (1*S*,2*S*,5*R*,6*S*)-2-[(2'*S*,3'*R*)-2-amino-3-hydroxy)-butyryl)amino-bicyclo[3.1.0]hexane-2,6-dicarboxylic acid;
h) (1S,2S,6R,6S)-2-[2'S-(2"S-amino-4-methylpentanonylamino)-propionylamino]-bicyclo[3.1.0]hexane-2,6-dicarboxylic acid; or
i) (1S,2S,5R,6S)-2-[2'(S)-(2"(S)-amino-propionylamino)-propionylamino]-bicyclo[3.1.0]hexane-2,6-dicarboxylic acid.

7. The compound of Claims 1, 2, 4, or 5 which is (1*S*,2*S*,5*R*,6*S*)-2-[(2'*S*)-(2'-amino-4'-methyl)-pentanoyl]aminobicyclo[3.1.0]hexane-2,6-dicarboxylic acid.

8. A pharmaceutically acceptable salt of a compound of formula I as claimed in Claims 1-7 which is an acid-addition salt made with an acid which provides a pharmaceutically acceptable anion or, for a compound which contains an acidic moiety, which is a salt made with a base which provides a pharmaceutically acceptable anion.

9. The pharmaceutically acceptable salt of a compound of formula I as claimed in Claim 8 wherein the salt is a hydrochloride salt.

10. The pharmaceutically acceptable salt of a compound of formula I as claimed in Claim 8 wherein the salt is a methane sulfonate salt.

11. A pharmaceutical formulation comprising in association with a pharmaceutically acceptable carrier, dilutent or excipient, a compound of formula I (or a pharmaceutically acceptable salt thereof) as provided Claims 1-10.

12. A process for preparing the compound of formula I, or a pharmaceutically acceptable salt thereof, as claimed in Claim 1 comprising:
(A) for a compound of formula I in which R¹³ and R¹⁴ are hydrogen, deprotecting a compound of formula IV 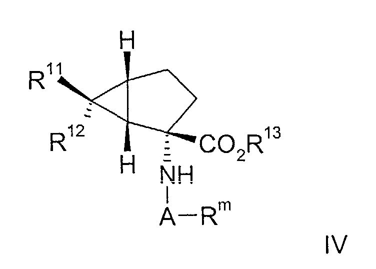 where R^{m} is an amine protecting group and R¹³ and R¹⁴ are carboxy protecting groups;
(B) for a compound of formula I in which R¹³ and R¹⁴ are both not hydrogen, acylating a compound of formula II 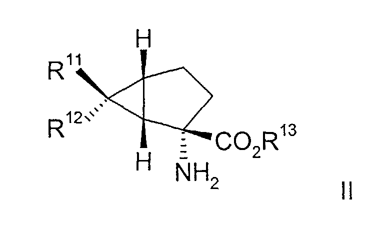 with a corresponding amino acyl of formula III
R^{m}A- III
in which R^{m} is an amine protecting group;
whereafter, for any of the above procedures, when a functional group is protected using a protecting group, removing the protecting group;
whereafter, for any of the above procedures, when a pharmaceutically acceptable salt of a compound of formula I is required, it is obtained by reacting the basic form of such a compound of formula I with an acid affording a physiologically acceptable anion, or, for a compound of formula I which bears an acidic moiety, reacting the acidic form of such a compound of formula I with a base which affords a pharmaceutically acceptable cation, or by any other conventional procedure.

13. A method for affecting the cAMP-linked metabotropic glutamate receptors in a patient, which comprises administering to a patient requiring modulated excitatory amino acid neurotransmission a pharmaceutically-effective amount of the compound of Claim 1.

14. A method for affecting the cAMP-linked metabotropic glutamate receptors in a patient, which comprises administering to a patient requiring modulated excitatory amino acid neurotransmission a pharmaceutically-effective amount of the compound of Claim 7.

15. A method for treating a neurological disorder in a patient which comprises administering to the patient in need of treatment thereof a pharmaceutically-effective amount of the compound of Claim 1.

16. The method of Claim 15 wherein said neurological disorder is cerebral deficits subsequent to cardiac bypass and grafting; cerebral ischemia; spinal cord trauma; head trauma; Alzheimer's Disease; Huntington's Chorea; amyotrophic lateral sclerosis; AIDS-induced dementia; perinatal hypoxia; hypoglycemic neuronal damage; ocular damage and retinopathy; cognitive disorders; idiopathic and drug-induced Parkinson's Disease; muscular spasms; migraine headaches; urinary incontinence; drug tolerance, withdrawal, and cessation; smoking cessation; emesis; brain edema; chronic pain; sleep disorders; convulsions; Tourette's syndrome; attention deficit disorder; and tardive dyskinesia.

17. The method of Claim 16 wherein said neurological disorder is drug tolerance, withdrawal, and cessation; or smoking cessation.

18. A method for treating a neurological disorder in a patient which comprises administering to the patient in need of treatment thereof a pharmaceutically-effective amount of a compound of Claim 7.

19. The method of Claim 18 wherein said neurological disorder is cerebral deficits subsequent to cardiac bypass and grafting; cerebral ischemia; spinal cord trauma; head trauma; Alzheimer's Disease; Huntington's Chorea; amyotrophic lateral sclerosis; AIDS-induced dementia; perinatal hypoxia; hypoglycemic neuronal damage; ocular damage and retinopathy; cognitive disorders; idiopathic and drug-induced Parkinson's Disease; muscular spasms; migraine headaches; urinary incontinence; drug tolerance, withdrawal, and cessation; smoking cessation; emesis; brain edema; chronic pain; sleep disorders; convulsions; Tourette's syndrome; attention deficit disorder; and tardive dyskinesia.

20. The method of Claim 19 wherein said neurological disorder is drug tolerance, withdrawal, and cessation; or smoking cessation.

21. A method for treating a psychiatric disorder in a patient which comprises administering to the patient in need of treatment thereof a pharmaceutically-effective amount of the compound of Claim 1.

22. The method of Claim 21 wherein said psychiatric disorder is schizophrenia, anxiety and related disorders, depression, bipolar disorders, psychosis, and obsessive compulsive disorders.

23. The method of Claim 22 wherein said psychiatric disorder is anxiety and related disorders.

24. A method for treating a psychiatric disorder in a patient which comprises administering to the patient in need of treatment thereof a pharmaceutically-effective amount of a compound of Claim 7.

25. The method of Claim 24 wherein said psychiatric disorder is schizophrenia, anxiety and related disorders, depression, bipolar disorders, psychosis, and obsessive compulsive disorders.

26. The method of Claim 25 wherein said psychiatric disorder is anxiety and related disorders.

27. A compound of formula IV 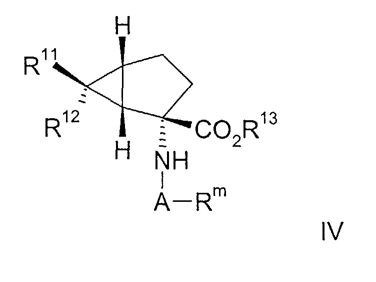 wherein R^{m} is an amine protecting group and the groups R¹¹, R¹² and R¹³ have any of the values defined in Claim 1.

28. A method of administering an effective amount of a compound of formula II, where R¹³ and R¹⁴ are both hydrogen (a di-acid), which comprises administering to a patient requiring modulated excitatory amino acid neurotransmission a pharmaceutically effective amount of a compound of Claim 1.

29. A novel compound of formula I substantially as hereinbefore described with reference to any of the Examples.

30. A method for affecting the CAMP-linked metabotropic glutamate receptors in a mammal, which comprises administering to a mammal requiring modulated excitatory amino acid neurotransmission a pharmaceutically effective amount of a compound of formula I substantially as hereinbefore described with reference to any of the Examples.

31. A process for preparing a novel compound of formula I substantially as hereinbefore described with reference to any of the Examples.
